# EUROPÄISCHE PATENTANMELDUNG

(11) **EP 3 025 702 A1**
(43) Veröffentlichungstag der Anmeldung: **01.06.2016**
(21) Anmeldenummer: 15196633.0
(22) Anmeldetag: 27.11.2015
(51) Int. Cl.: A61K 8/25, A61Q 17/04, C01B 33/027, C01B 33/029

(54) **HÖCHSTREINE, AMORPHE SILICIUMPULVER, VERFAHREN ZU DEREN HERSTELLUNG ALS AUCH DEREN VERWENDUNG**

(30) Priorität: 28.11.2014 EP 14195304
(71) Anmelder: Evonik Degussa GmbH, 45128 Essen (DE)
(72) Erfinder: LANG, Jürgen Erwin, 76229 Karlsruhe (DE); RAULEDER, Hartwig, 79618 Rheinfelden (DE); UEHLENBRUCK, Goswin, 61440 Oberursel (DE)

(57) **Zusammenfassung**

Die Erfindung betrifft höchstreine, nanopartikuläre, amorphe Siliciumpulver, die vorzugsweise mit Elektronendonatoren- und/oder Elektronenakzeptoren legiert sein können. Ferner wird ein Verfahren zur Herstellung der Siliciumpulver sowie die Verwendung eines Reaktors zur Herstellung der Siliciumpulver offenbart. Die erfindungsgemäßen Siliciumpulver können vorzugsweise zur Herstellung von Halbleiterausgangsstoffen, Halbleitern, Thermoelementen zur Energierückgewinnung aus Abwärme, insbesondere von hochtemperaturstabilen Thermoelementen verwendet werden.

## Beschreibung

Die Erfindung betrifft höchstreine, nanopartikuläre, amorphe Siliciumpulver, die optional mit Elektronendonatoren- und/oder Elektronenakzeptoren legiert sein können. Ferner wird ein Verfahren zur Herstellung der Siliciumpulver sowie die Verwendung eines Reaktors zur Herstellung der Siliciumpulver offenbart. Die erfindungsgemäßen Siliciumpulver können vorzugsweise zur Herstellung von Halbleiterausgangsstoffen, Halbleitern, Thermoelementen zur Energierückgewinnung aus Abwärme, insbesondere von hoch-temperaturstabilen Thermoelementen verwendet werden.

In der Solar- und Halbleiterindustrie sowie der LED- und Display-Herstellung besteht ein großer Bedarf an hochreinen Verbindungen, die zur Herstellung und Nutzung der Technologien benötigt werden.

Den bekannten Prozessen zur Herstellung von Silicium gemeinsam sind hohe Rohstoffkosten, ein hoher Energieeintrag und eine für die vorgenannten technischen Anwendungsfelder nicht ausreichende Reinheit der erhaltenen Produkte.

Vielen der heutigen Industrieapplikationen von Silicium sind in der Regel die sehr hohen Reinheitsanforderungen gemeinsam. Daher darf die Verunreinigung der umzusetzenden Silane oder Halogensilane höchstens im Bereich von wenigen mg/kg (ppm-Bereich) und für spätere Anwendungen in der Halbleiterindustrie im Bereich von wenigen µg/kg (ppb-Bereich) liegen.

Bislang wurden Verfahren entwickelt, deren Ziel die Herstellung von Einkristallen oder polykristallinen Materialien war, um die Reinheitsanforderungen über die Kristallisation einzustellen. Ein Nachteil von kristallinen Materialien ist, dass der Energieeintrag zur Rekristallisation höher ist als bei einer Bereitstellung von amorphem hoch- oder höchstreinen Silicium enthaltenden Materialien. Anschließend können die amorphen Materialien ökonomischer aufgeschmolzen werden und zur Kristallisation von kristallinem Silicium eingesetzt werden.

Aufgabe war die Bereitstellung eines Verfahrens zur einfachen und ökonomischen Gewinnung von hochreinem Silicium, das erfindungsgemäß nicht kristallin oder polykristallin vorliegen sollen. Aufgabe der Erfindung war die Herstellung von amorphen, hochreinen bis höchstreinen Silicium enthaltenden Partikeln, um den Energieeintrag bei einer möglichen späteren Kristallisation oder anderweitigen Weiterverarbeitung durch Aufschmelzen zu minimieren. Des Weiteren bestand die Aufgabe einen Prozess zu finden, der kontinuierlich oder satzweise hohe Durchsätze der herzustellenden Verbindungen erlaubt. Eine weitere Aufgabe bestand darin die Partikel darüber hinaus definiert mit Elektronenakzeptoren oder Elektronendonatoren zu versehen, die das technische Anwendungsprofil der Silicium enthaltenden Partikel deutlich erweitert. So sollen Silicium enthaltende Partikel hergestellt werden, die in Thermoelementen oder in hochtemperaturstabilen Folien, Schichten, Kabelbändern oder Bauteilen als umweltfreundliche Verbindungen zum Einsatz kommen können. So soll ein Verfahren entwickelt werden, dass auch ohne die Verwendung von Tetrachlorsilan wirtschaftlich ist.

Des Weiteren sollen die spektrale Eigenschaften der Silicium enthaltenden Partikel über die Verteilung der Elektronenakzeptoren und/oder Elektronendonatoren im jeweiligen Partikel und/oder auf dem jeweiligen Partikel einstellbar sein. Beispielsweise sollen die, wärmeleitenden Eigenschaften für die Thermoelektrik verringert werden oder im Zusammenhang mit einer verbesserten Transparenz sollen die wärmeleitenden Eigenschaften verbessert werden, um Wärme besser aus elektronischen Geräten abzuleiten. Das erfindungsgemäße Verfahren soll zu dem wirtschaftlicher sein als bekannte Verfahren, in dem die nicht umgesetzten Reaktionsprodukte oder die verwendeten inerten Gase ökonomischer wiederverwendet werden können.

Die Aufgaben konnten in überraschender Weise dadurch gelöst werden, dass die oben genannten Siliciumverbindungen durch Umsetzung von Monosilan oder höheren H-Silanen, wie der Formel MeHn oder Me₂H₂ₙ₋₂, wobei Me für Silicium und n für eine ganze Zahl steht, Halogensilanen, höheren Halogensilanen oder Alkoxysilanen mit C oder N oder Ge enthaltenden Spezies in einem thermischen Prozess, ggf. kombiniert mit einem Plasmaprozess oder in einem reinen Plasmaprozess umgesetzt werden. Höhere H-Silane oder Chlorsilane werden auch als Polysilane oder Polychlorsilane bezeichnet. Die höheren H-Silane oder höheren Chlorsilane werden vor Ihrer Umsetzung in die Gasphase überführt. Das Halogen kann ausgewählt sein aus Fluor, Chlor, Brom oder Iod, bevorzugt ist Chlor.

Gelöst werden die Aufgaben durch das erfindungsgemäße Siliciumpulver nach Anspruch 1 und das erfindungsgemäße Verfahren nach Anspruch 6 und durch Verwendung eines Freiraum-Infrarotstrahlungs-Reaktors nach Anspruch 14. Bevorzugte Ausführungsformen sind in den Unteransprüchen und in der Beschreibung erläutert.

Erfindungsgemäß wird ein großtechnisches, industrielles, vorzugsweise kontinuierliches Verfahren zur Herstellung von Siliciumpulvern umfassend Primärpartikel und optional Cluster der Primärpartikel bereitgestellt. Bevorzugte Siliciumpulver umfassen Silicium-Pulver, die vorteilhaft auch mit Elektronenakzeptoren- oder Elektronendonatoren beschichtet sein können. Die Umsetzung, insbesondere umfassend die Zersetzung und Bildung der Siliciumpartikel oder der beschichteten Siliciumpartikel, kann bei Temperaturen ab 150 °C, bevorzugt ab 400 bis 1500 °C zur Herstellung amorpher Pulver erfolgen. Zur Herstellung amorpher Siliciumpulver werden kurze Kontaktzeiten, vorzugsweise bei Temperaturen unter 1300 °C, insbesondere um 1100 °C gewählt. Die Abscheidung der Siliciumpulver erfolgt in einer kühleren Zone des Reaktors. Bevorzugte Kontaktzeiten zur Zersetzung und Abscheidung der Primärpartikel liegen von 0,010 bis 600 Millisekunden.

Die Aufgaben konnten auch in einer Alternative überraschend gelöst werden durch eine Behandlung im nicht-thermischen Plasma, insbesondere in einem nicht-thermischen Plasma erzeugt durch eine Gasentladung, auf einen die erforderlichen Reaktionskomponenten enthaltenden Massestrom erreicht werden.

Gegenstand der Erfindung ist die Erzeugung von hochreinen, gegebenenfalls mit Donatoren oder Akzeptoren beschichteten und/oder legierten Siliciumpulvern unter thermischen Bedingungen sowie auch die Gewinnung von hochreinen, insbesondere höchstreinen, gegebenenfalls legierten Silicium Pulvern durch Reaktion im Plasma.
So benötigen übliche konventionelle Verfahren, die auf einer Umsetzung von SiCl₄, basieren von 40 und mehr als einhundert kWh/kg. Die erfindungsgemäßen Verfahren beruhen vorzugsweise auf einer Umsetzung von Monosilan mit einem Energiebedarf von weniger als 40 kWh/kg. Des Weiteren wird für die Umsetzung im Plasma der Energiebedarf weiter reduziert auf kleiner 1,4 kWh/kg. Die Erfindung betrifft auch ein Verfahren zur Gewinnung von hochreinen, mit (Elektronen-) Donatoren oder (Elektronen-)Akzeptoren legierten Silicium Primärpartikeln und optional Clustern der Primärpartikeln.

Gegenstand der Erfindung sind Siliciumpulver umfassend im Wesentlichen amorphe Primärpartikel, vorzugsweise weisen die Siliciumpulver eine Kristallinität von kleiner gleich 15 % auf, insbesondere kleiner gleich 5 %, bevorzugt kleiner gleich 2 % auf, insbesondere bis zur Nachweisgrenze, bevorzugt bis 0,5 %, weiter bevorzugt bis 0,1%.

Die Kristallinität kann mittels XRD bestimmt werden (Referenzspektrum: size fraction 15% kristallin, 85 % amorph, bestimmt durch Einwaage von SiO₂ als Referenzspektrum: 15 + 85 %).
Alternativ oder zusätzlich kann die Kristallinität mittels XRPD ermittelt werden über die Formel %Kristallinität = (100 x A)/(A + B -C) mit A = gesamte Peakfläche der Reflexe der kristallinen Bestandteilen des Diffraktogramms; B = gesamte Fläche unterhalb der Peakfläche A; C = luftstreuend-, fluoreszierend- und gerätebedingte Untergrundfläche. Gesamte Peakfläche A mit Untergrund genau unterhalb der schmalen Reflexe der Si-Phase. Die Untergrundfläche C wurde anhand der XRD-Diffraktorgramme des Si-Referenzstandards NIST 640 (Si-Standard = 100% Kristallinität) ermittelt. Fläche B entspricht einem eingelegten Untergrundverlauf sowie dem konstanten Untergrund C. Berechnung (HighScore Plus Software). Ein Kristall ist ein anisotroper, homogener Körper, der eine dreidimensional periodische Anordnung der Bausteine besitzt und ein XRPD mit klar definierten auflösbaren Reflexen besitzt.

Gegenstand der Erfindung ist ein Siliciumpulver, wobei das Siliciumpulver Primärpartikel mit einer Partikelgröße von 1 nm bis 1000 nm aufweist, insbesondere von 1 bis 100 nm, vorzugsweise 5 bis 60 nm, besonders bevorzugt von 5 bis 50 nm, weiter bevorzugt von 5 bis 25 nm, und die Partikel im Wesentlichen amorph sind. Besonders bevorzugte Siliciumpulver umfassen Primärpartikel, die je nach Verfahrensführung definierte Partikelgrößen aufweisen können, wobei besonders bevorzugte Primärpartikelgrößen von 2 bis 100 nm aufweisen, bevorzugt von 2 bis 50 nm, besonders bevorzugt sind Primärpartikelfraktionen mit Partikelgrößen um 10 nm, 20 nm, 30 nm, 40 nm, alternativ um 30 bis 50 nm, 40 nm jeweils unabhängig mit einer Abweichung von plus/minus 10 nm, bevorzugt plus/minus 5 nm.

Als besonders bevorzugt gelten Siliciumpulver in denen weniger als 50 % der Partikel eine Abweichung von der mittleren Korngröße d₅₀ und weniger als 25 % eine Abweichung von größer gleich 50 % von der mittleren Korngröße d₅₀ aufweisen.

Bevorzugt sind weiterhin Pulver, deren Primärpartikel einen mittleren Durchmesser d₅₀ (gemessen durch TEM-Auswertung; TEM = Transmissions-Elektronenmikroskop) im Bereich von 5 bis 170 nm, vorzugsweise von 30 bis 70 nm, aufweisen.

Ob die Teilchen sich sphärisch oder in der Form von Whiskern ausbilden hängt unter anderem von der H₂-Konzentration bei der Darstellung ab. Je nach Temperaturprofil, Verdünnungsgas (Konzentration, Strömungsgeschwindigkeit) Herstellbedingungen können Primärpartikel isoliert werden oder überwiegend Primärpartikel, die zu Clustern agglomeriert sind, erhalten werden. Ihre Herstellung erfolgt bevorzugt um 100 °C sowie bei bis zu 700°C +/- 100 °C im kalten Plasma. (Das kaltes Plasma wird mittels transienter Hochspannungsentladung in einer bipolaren Elektrodenanordnung (Nullpotential, Hochspannungselektrode), die additiv mit einer dielektrischen Barriere ausgestattet sein kann, erzeugt. Über Die bevorzugten Betriebsparameter für eine planare Elektrodenanordung mit einer Gasschlagweite (GAP) von rund 4 mm sind 13 kVp am Gasraum anliegendes Potential mit einer Impulsdauer von gerundet 950 Nanosekunden und einer Wiederholrate von gerundet 8 000 pro Sekunde. Ein typischer spezifischer Prozessgasflow mit ca. 1 % Monosilan in Argon Matrix liegt gerundet bei 42 cm/s (Volumenflow 2400 cm³/min geteilt durch 1 cm² Elektrodenfläche).

Gegenstand der Erfindung sind auch Siliciumpulver umfassend agglomerierte Cluster der Primärpartikel, insbesondere weisen die Cluster eine Größe von 10 nm bis 3 µm auf, vorzugsweise von 500 nm bis 3 µm.

Besonders bevorzugt umfassen die Siliciumpulver mindestens einen Primärpartikel mit einer Siliciumlegierung mit größer gleich 10²¹ Atomen/cm³, insbesondere bis 10²² Atomen/cm³ ausgewählt aus mindestens einem Elektronendonator und Elektronenakzeptor.

Gleichfalls Gegenstand der Erfindung sind Siliciumpulver und ein Verfahren zur Herstellung dieser, indem Siliciumpulver umfassend Primärpartikel erhalten werden, die mindestens einen Primärpartikel mit einer Siliciumlegierung umfassen mit größer gleich 10²¹ Atomen/cm³, insbesondere größer gleich 10²¹ Atomen/cm³ bis 100 % Atomen/cm³ (gleichbedeutend bis 6,2 · 10²² Atomen/cm³ im Fall von Bor),wobei die Atome ausgewählt sind aus mindestens einem Elektronendonator und Elektronenakzeptor. Weiter bevorzugt sind Siliciumlegierungen mit größer gleich 1 Promille, bevorzugt von 1 bis 99% Atomen/cm³ legiert, besonders bevorzug 0,1 bis 10% Atomen/cm³.

Ferner sind Gegenstand der Erfindung Siliciumpulver umfassend Primärpartikel und optional Cluster der Primärpartikel: a) die jeweils unabhängig aus einer Siliciumlegierung bestehen und/oder b) die jeweils unabhängig mindestens eine intrinsische Zone einer Siliciumlegierung mit mindestens einem Elektronendonator oder mindestens einem Elektronenakzeptor mindestens teilweise aufweisen und/oder c) jeweils unabhängig mindestens eine Zone einer Siliciumlegierung umfassend einen Elektronendonator, Elektronenakzeptor, eine einen Elektronendonator oder Elektronenakzeptor enthaltenden Verbindung als eine mindestens teilweise Beschichtung aufweisen. Dabei ist es besonders bevorzugt, wenn die Zone(n) als äußere Beschichtung(en) auf den Submicropartikeln und optional Macropartikeln vorliegen. Weiter bevorzugt sind die sind die Elektronendonatoren und -akzeptoren ausgewählt aus einem Element der Gruppe 13 oder 15 des Periodensystems der Elemente nach IUPAC, bevorzugt Bor, Aluminium, Phosphor, Arsen und/oder Antimon.

Ebenso ist Gegenstand der Erfindung ein Siliciumpulver umfassend Primärpartikel und optional Cluster einer Siliciumlegierung mit einem Element der Gruppe 13 Gruppe 15 des Periodensystems der Elemente nach IUPAC oder 15 und/oder mindestens umfassend eine Zone einer Siliciumlegierung mit einem Element der Gruppe 13 oder 15, insbesondere sind sie mit einer Zone der Siliciumlegierung mindestens teilweise beschichtet, vorzugsweise im Wesentlichen vollständig beschichtet.

Besonders bevorzugt sind Siliciumpulver umfassend a) Primärpartikel, die aus einer Siliciumborlegierung bestehen, und/oder b) die Primärpartikel umfassen mindestens eine intrinsische Zone einer Siliciumborlegierung, und/oder c) die Primärpartikel umfassen mindestens eine Zone einer Siliciumborlegierung als eine zumindest teilweise Beschichtung der Primärpartikel.

Gleichfalls werden Siliciumpulver beansprucht, deren Primärpartikel Cluster ausbilden, in denen mindestens zwei Partikel an ihren Oberflächen aneinander geschmolzen sind. Diese Cluster können als lineare Ketten, in Form von Drähten oder auch verzweigt vorliegen.

Ferner werden nach einer Alternative Siliciumpulver beansprucht, deren Primärpartikel mit Bor legiert sind, indem sie eine zumindest teilweise oder vollständige Bor enthaltende intrinsische Zone oder eine äußere Zone als zumindest teilweise Beschichtung aufweisen.

Entsprechend einer Alternative umfassen die Siliciumpulver vorzugsweise Partikel, die im Wesentlichen einen sphärischen Habitus aufweisen. Die mittlere Sphärizität, definiert als Aspektverhältnis der Durchmesser der im 90° Winkel aufeinander stehenden Durchmesser, ist vorzugsweise kleiner gleich 1,6, vorzugsweise kleiner gleich 1,4 bis größer gleich 0,9, vorzugsweise von 0,95 bis 1,2, weiter bevorzugt von 0,6 bis 1,4. Das Aspektverhältnis nahe der Kugelform erlaubt eine ideale Homogenisierung und/oder gute Verpressbarkeit von Sinterrohlingen an den Kontaktpunkten und damit einen besseren Wärmeübergang.

Entsprechend einer weiteren bevorzugten Ausführungsform sind die Partikel der Siliciumpulver im Wesentlichen nicht porös und weisen daher im Wesentlichen keine innere Oberfläche auf.

Um die elektrischen Eigenschaften der Siliciumpulver auf die jeweilige Anforderung einstellen zu können, ist es bevorzugt, wenn die Partikel eine zumindest teilweise oder vollständige Beschichtung mit einer Siliciumborlegierung aufweisen. Die Schichtdicke der Beschichtung kann 1 nm bis 100 nm, insbesondere 1 bis 20 nm betragen, besonders bevorzugt sind 1 bis 5 und 3 bis 10 nm.

Bevorzugte Siliciumpulver umfassen Partikel von größer gleich 56,9 Gew.-% Silicium bevorzugt mit einem Gehalt von größer gleich 76,9 Gew.-%, besonders bevorzugt mit einem Gehalt von größer gleich 95 Gew.-%. Alternativ können die Silicium-Partikel im Wesentlichen ohne äußere Matrix oder Beschichtung vorliegen, die auch eine passivierende Oxidschicht umfassen kann. Bevorzugt ist dann ein Siliciumgehalt von größer gleich 98 Gew-% in Fall der dotierten und/oder legierten Partikel, größer gleich 99,99 Gew-% für die reinen Siliciumpartikel. Nach einer Alternative liegen die Silicium-Partikel mit einer Siliciumdioxid-Schicht (Core-Shell) von typisch 1 nm vor. Ferner umfassen die Siliciumpulver Partikel entsprechend mit Germanium, Bor, Phosphor, Arsen, Selen und/oder Kohlenstoff et cetera legierte Siliciumpulver, insbesondere entsprechend legierte Partikel von Silicium mit Bor oder Phosphor.

Dabei ist es erfindungsgemäß besonders bevorzugt, wenn die Siliciumpulver oder deren Legierung hochrein sind, insbesondere höchstrein. Als hochrein gilt ein Siliciumpulver, wenn Silicium mit einem Gehalt größer gleich 99,9999 Gew.-% in der Gesamtzusammensetzung vorliegt, bevorzugt mit einem Gehalt von größer gleich 99,99999 Gew.-%. Als höchstrein gilt ein Siliciumpulver mit einem Gehalt an Silicium von größer gleich 99,999999 Gew.-% in der Gesamtzusammensetzung.

Die Verunreinigungen in den jeweiligen Edukten und Verfahrensprodukten werden mittels dem Fachmann bekannter Probenaufschlussverfahren bspw. durch Nachweis im ICP-MS (Analytik für die Bestimmung der Spurenverunreinigung) bestimmt.

Das hochreine bis höchstreine Siliciumpulver mit einem Gehalt an Silicium von größer gleich 99,99 Gew.-% kann ein Verunreinigungsprofil aufweisen an Bor, Phosphor, Arsen, Aluminium, Eisen, Natrium, Kalium, Nickel, Chrom, Schwefel, Barium, Zirkon, Zink, Titan, Calcium, Magnesium, Kupfer, Chrom, Cobalt, Zink, Vanadium, Mangan und/oder Blei von unter 100 ppm, bevorzugt von unter 20 ppm bis 0,001 ppt, besonders bevorzugt von 10 ppm und 0,001 ppt in Bezug auf die hochreine Gesamtzusammensetzung an Siliciumpulver auf.

Zur Herstellung der hoch- oder höchstreinen Siliciumpulver wird ein hoch- bis höchstreines Silan eingesetzt, wobei die Definition von rein bis höchstreines Silan mit Halbleiterqualität verwendet werden, wie monomeres und/oder polymeres Monosilan/-, H-Silan und/oder Chlorsilan eingesetzt, insbesondere der allgemeinen Formel I, II und/oder III oder eine Mischung der enthaltend, wie höchstreines Tetrachlorsilan, höchstreines Trichlorsilan und/oder höchstreines Dichlorsilan, vorzugsweise mit einem Gehalt an einem Silan von 80 bis 99,9999999 Gew.-%, ad 100 Gew.-% ggf. Polysilane, und mit einer Gesamtverunreinigung von kleiner gleich 100 Gew.-ppm bis 0,001 Gew.-ppt als hochreines Silan, vorzugsweise kleiner 50 Gew.-ppm bis 0,001 Gew.-ppt als höchstreines Silan, bevorzugt kleiner gleich 40 Gew.-ppm bis 0,001 Gew.-ppt an Gesamtverunreinigung mit den nachstehend genannten Elementen. Alternativ kann statt einem einzelnen Silan auch eine Mischung von Silanen eingesetzt werden, sofern sie dem vorgenannten Anforderungsprofil an den Gehalt des Silans entspricht.

In den erfindungsgemäßen Siliciumpulvern weisen die Primärpartikel und optional die Cluster in der Gesamtzusammensetzung einen Gehalt von kleiner gleich 2 % Atome/cm³, vorzugsweisen kleiner gleich 2000 ppm-Gew Sauerstoff auf, vorzugweise kleiner gleich 1000 ppm-Gew, insbesondere kleiner 10 ppm-Gew., bevorzugt kleiner gleich 1 ppb-Gew bis zur Nachweisgrenze, bspw. bis 1 ppt-Gew.-%.

Der Gehalt an Verdünnungsgasen, wie Xenon, Argon, Krypton, oder auch Stickstoff (ausgenommen im Fall der gewünschten Herstellung der Halbmetallnitride) beträgt in der Gesamtzusammensetzung der Halbmetallpulver, bspw. des Silicumpulver kleiner 1% Atome/cm³, insbesondere kleiner gleich 1000 ppm-Gew., 10 ppm-Gew., 1 ppb-Gew bis zur Nachweisgrenze, bspw. bis 1 ppt-Gew.

Die Analyse erfolgt neben ICP-MS und HP-GD-MS (High Purity Glow Discharge Mass Spectroskopy) bevorzugt mittels Neutronenaktivierungsanalyse (NAA). Die NAA ist eine hochempfindliche physikalische Technik der analytischen Chemie zur qualitativen und quantitativen Spurenanalyse, bei der die zu untersuchende Probe mit Neutronen (oder anderen Teilchen) beschossen wird.

Bei der NAA wird eine Probe von nur wenigen Milligramm (oder Fallweise wenige µg) dem Neutronenstrom (eines Kernreaktors) ausgesetzt. Die Neutronen reagieren mit den Kernen der Probe und machen aus den stabilen Isotopen radioaktive Isotope, deren Massenzahl eins höher ist als die Massenzahl des stabilen Isotops. Bei diesem ersten Kernprozess wird ein promptes γ - Quant ausgesandt, dessen Energie man messen kann und das Auskunft über den Ausgangskern gibt. In den meisten Untersuchungen wird aber erst der Zerfall des entstandenen radioaktiven Kerns zur Analyse verwendet. Es zerfällt anschließend mit der für ihn typischen Halbwertzeit unter Aussendung eines Betateilchens und charakteristischer Gammastrahlung, die in einem Gammaspektrometer untersucht wird. Auf diese Weise sind praktisch alle vorkommenden Elemente in einer Probe quantitativ und qualitativ nachweisbar. Aus den bekannte Eigenschaften der Atomkerne lassen sich neben den Gehalt der Elemente, sogar ihrer Isotope bestimmen. Die Messungen können bspw. am Berlin Neutron Scattering Center durchgeführt werden.

Die Nachweisgrenzen für die Bestimmung von Xenon (Xe), Argon (Ar), Krypton (Kr), oder auch Stickstoff durch Neutronenaktivierung bei einer Bestrahlzeit von 1 Stunde bei einer sogenannten Flußdichte an thermischen Neutronen von 10¹⁴ cm⁻² s⁻¹ liegen bei ca. 10⁻⁸ g (Ne, Xe), ca. 10⁻⁹ (Kr), ca. 10⁻¹⁰ (Ar) und ca. 10⁻⁵ g für Sauerstoff (O₂). Die Nachweisgrenze kann damit insbesondere für kleine Probenmengen kleiner gleich 1000 ppm-Gew., 10 ppm-Gew., 1 ppb-Gew bzw. bis zur Nachweisgrenze liegen, bspw. bis 1 ppt-Gew. betragen bspw. bis 1 ppt-Gew.

Eine Zone in Sinne der Erfindung umfasst definierte dreidimensionale Bereiche in den Partikeln, bevorzugt sind Zonen, die dem Kern des Partikels entsprechen und mindestens eine äußere Beschichtung entspricht einer weiteren Zone. Der Kern des Partikels kann bis zu 95 Volumen-%, insbesondere bis zu 99,5 Vol.-% des Partikels mit Beschichtung entsprechen. Die Beschichtung entspricht dann 0,5 bis 5 Vol.-% des Gesamtpartikels umfassend die Beschichtung.

Das reine Siliciumpulver mit einem Gehalt an Silicium von größer gleich 95 Gew.-% kann ein erweitertes Legierungsprofil aufweisen an Bor, Phosphor, Arsen, Aluminium, Antimon, Selen, Eisen, Natrium, Kalium, Nickel, Chrom, Schwefel, Barium, Zirkon, Zink, Titan, Calcium, Magnesium, Kupfer, Chrom, Cobalt, Zink, Vanadium, Mangan und/oder Blei von unter 2 % bis **1** ppb, bevorzugt von 2 % und 0,1 % (Atome/cm³) in Bezug auf die Gesamtzusammensetzung bspw. des Siliciumpulvers.

Entsprechend einer besonders bevorzugten Ausführungsform weist das Siliciumpulver, insbesondere die Partikel des Siliciumpulvers, thermoelektrische Eigenschaften auf. So können diese Siliciumpulver mit thermoelektrischen Eigenschaften vorzugsweise in einem thermoelektrischen Generator verwendet werden. Besonders bevorzugt ist eine Siliciumpulver umfassend mindestens eine Siliciumlegierung.

Entsprechend einer vorzugsweisen Ausführungsform ist das Siliciumpulver ein Verbindungshalbleiter. Übliche Thermoelemente aus Metallen wandeln thermische Energie nur sehr ineffizient in elektrische Energie um. Mit den erfindungsgemäßen Partikeln mit thermoelektrischen Eigenschaften können auf einfache und wirtschaftliche Weise thermoelektrische Partikel in einem großtechnischen Verfahren hergestellt werden. Eine wirtschaftliche Beschichtung von Oberflächen, die mit heißen Komponenten, Oberflächen, heißen Dämpfen oder Gasen in Kontakt kommen ist mit den erfindungsgemäßen Partikeln oder daraus hergestellten Beschichtungen möglich. Somit gelingt es mit Hilfe der thermoelektrischen Partikel aus Abwärme elektrische Energie zu gewinnen. Besonders bevorzugte Anwendungsfelder bieten Verbrennungsmotoren und die mit den Abgasen in Kontakt kommenden Vorrichtungen, insbesondere deren Oberflächen, Gas- oder Dampfturbinen sowie die damit verbauten Vorrichtungen, Flugzeugturbinen oder auch Batterien, die große Hitze entwickeln.

Ebenso Gegenstand der Erfindung ist ein Verfahren zur Herstellung von Siliciumpulver sowie Siliciumpulver erhältlich nach diesem Verfahren, indem
a) mindestens eine bei erhöhter Temperatur gasförmige Siliciumverbindung, nach einer Alternative optional in Gegenwart eines bei erhöhter Temperatur reaktiven Gases, und
b) optional in Gegenwart eines Verdünnungsgases, insbesondere eines unter den Verfahrensbedingungen inerten Verdünnungsgases, in einer im Wesentlichen sauerstofffreien Atmosphäre unter (i) thermischen Bedingungen und/oder (ii) im Plasma zersetzt wird, und in Form eines Siliciumpulvers erhalten wird mit Primärpartikel deren Partikelgröße von 1 nm bis 100 nm liegt, vorzugsweise von 1 bis 50 nm, insbesondere werden die Siliciumpulver abgeschieden, wobei die Partikel, insbesondere die Primärpartikel, im Wesentlichen amorph sind.

Als im Wesentlichen amorph gilt ein Pulver, das röntenamorph ist. Als röntgenamorph gilt vorzugsweise ein Pulver mit einem Kristallinität kleiner 2,96 %. Wobei der Kristallinitätsgrad nach folgender Methode mittels XRPD ermittelt werden über die Formel %Kristallinität = (100 x A)/(A + B -C) mit A = gesamte Peakfläche der Reflexe der kristallinen Bestandteilen des Diffraktogramms; B = gesamte Fläche unterhalb der Peakfläche A; C = luftstreuend-, fluoreszierend- und gerätebedingte Untergrundfläche. Gesamte Peakfläche A mit Untergrund genau unterhalb der schmalen Reflexe der Si-Phase. Die Untergrundfläche C wurde anhand der XRD-Diffraktogramme des Si-Referenzstandards NIST 640 (Si-Standard = 100% Kristallinität) ermittelt. Fläche B entspricht einem eingelegten Untergrundverlauf sowie dem konstanten Untergrund C. Berechnung (HighScore Plus Software).

Gegenstand der Erfindung sind Siliciumpulver erhältlich nach dem Verfahren umfassend im Wesentlichen amorphe Primärpartikel, insbesondere weisen die Siliciumpulver, vorzugsweise die Primärpartikel, eine Kristallinität von kleiner gleich 15 % auf, insbesondere kleiner gleich 5 %, bevorzugt kleiner gleich 2 % auf, vorzugsweise bis zur Nachweisgrenze, bevorzugt bis größer gleich 0,5 %, weiter bevorzugt bis größer gleich 0,1% auf. Somit sind größer gleich 85 % bis kleiner gleich 99,9% der Siliciumpulver amorph, vorzugsweise größer gleich 95%, bevorzugt größer gleich 98%. Der Grad der Kristallinität also der Anteil an amorphem Metallverbindungspulver kann über das erfindungsgemäße Verfahren definiert eingestellt werden.

In röntgenamorphen Pulvern gibt es im XRPD keine scharfen, sondern nur wenige diffuse Interferenzen bei kleinen Beugungswinkeln. Stoffe mit einem derartigen Röntgenbeugungsdiffraktorgramm bezeichnet man als *röntenamorph*.

Kristallin: Kristall ist ein anisotroper, homogener Körper, der eine dreidimensional periodische Anordnung der Bausteine besitzt und ein XRPD mit klar definierten auflösbaren Reflexen besitzt. Gegenstand der Erfindung ist auch ein Verfahren in dem Siliciumpulver umfassend Primärpartikel mit einer Siliciumlegierung mit größer gleich 10²¹ Atomen/cm³ ausgewählt aus mindestens einem Elektronendonator und Elektronenakzeptor erhalten werden.

Gegenstand der Erfindung ist auch ein Verfahren zur Herstellung der Siliciumpulver umfassend mindestens den Schritt (I) Zersetzung (A) der Siliciumverbindung, optional im Gemisch mit einem Verdünnungsgas, unter (i) thermischen Bedingungen und/oder (ii) im Plasma, und Ausbildung von Primärpartikeln und/oder Clustern der Primärpartikel innerhalb von 10 bis 1000 Millisekunden, insbesondere nach einer Alternative innerhalb von 10 bis 100 Millisekunden, vorzugsweise innerhalb von 20 bis 80 Millisekunden, bevorzugt um 60 Millisekunden, vorzugsweise mit plus/minus 5 Millisekunden, und nach einer Alternative innerhalb von 50 bis 1000 Millisekunden, insbesondere innerhalb von 100 bis 500 Millisekunden erfolgt, bevorzugt kleiner gleich 100 bis 400 Millisekunden. Dabei ist es besonders bevorzugt, wenn die Temperatur von 1000 bis 1400 °C, vorzugsweise von 1100 bis 1300 °C mit einer Schwankung von plus/minus 50 °C beträgt. Die Vorstehenden Angaben in Millisekunden entsprechen vorzugsweise der Verweilzeit/ Zeitspanne im Reaktionsraum. Nach einer Verfahrensalternativen kann die Verweilzeit auch die Zeit zur Bildung der Zonen, wie der intrinsischen Zonen und/oder Beschichtung mitumfassen und nach einer anderen Alternative entspricht die Verweilzeit der Zeitspanne ohne Legierung und/oder Beschichtung der Partikel oder Cluster. Alternativ kann zu einem definierten Zeitpunkt eine Beschichtung der Partikel durch die Gegenwart eines reaktiven Gases erfolgen, dessen Zersetzungsprodukte sich auf den Primärpartikeln und Clustern als Beschichtung abscheiden.

Bevorzugt erfolgt die thermische Zersetzung in den Schritten a) eine gasförmige Siliciumverbindung wird optional b) in Gegenwart mindestens eines Verdünnungsgases unter (i) thermischen Bedingungen bei einer Temperatur von 600 bis 1500 °C zersetzt, insbesondere von 1000 bis 1500 °C, vorzugsweise unter 1300 °C. Besonders bevorzugt ist eine Zersetzung bei Temperaturen um 1000 bis 1300 °C, besonders bevorzugt um 1250 °C mit plus minus/ 50 °C, insbesondere innerhalb von 10 bis 100 Millisekunden, bevorzugt innerhalb von 20 bis 80 Millisekunden, besonders bevorzugt innerhalb von 20 bis 60 Millisekunden, vorzugsweise um 40 Millisekunden. Die Abkühlung zur Abscheidung der Siliciumpulver umfassend die Primärpartikel erfolgt vorzugsweise unmittelbar anschließend auf eine Temperatur unter 1000 °C, vorzugsweise unter 800 °C, bevorzugt auf unter 600 °C innerhalb eines Zeitraumes von kleiner gleich 600 Millisekunden, insbesondere kleiner gleich 40 Millisekunden, bevorzugt innerhalb kleiner gleich 20 Millisekunden im Fall einer kontinuierlichen Fahrweise. Bei Satzweiser Fahrweise können die Verweilzeiten deutlich höher bspw. um Faktor 100 höher liegen.

Erfindungsgemäß wird hochreines Monosilan optional in Gegenwart eines Verdünnungsgases unter thermischen Bedingungen zersetzt. Vorteilhaft kann auch eine Gasphasenabscheidung von Methylsilan bei 1000 bis 1500 °C erfolgen, bevorzugt um 1000 bis 1300 °C, insbesondere mit einer Abkühlung auf unter 800 °C innerhalb von 200 Millisekunden vorzugsweise innerhalb weniger 100 Millisekunden.

Besonders bevorzugt ist eine Verfahrensalternative, in der mindestens eine Siliciumverbindung und optional in Gegenwart mindestens eines Verdünnungsgases unter (i) thermischen Bedingungen bei einer Temperatur von 600 bis 1400 °C zersetzt wird, vorzugsweise von 1000 bis 1300 °C, besonders bevorzugt von 1100 °C bis kleiner gleich 1300 °C plus/minus 50 °C, wobei überwiegend amorphe Silicium Primärpartikel und optional Cluster der amorphen Primärpartikel isoliert werden können. Ein bevorzugtes im Verfahren durchlaufenes Temperaturprofil umfasst Temperaturen größer gleich 600 °C bis kleiner gleich 1100 °C und anschließend kleiner gleich 700 °C. Wobei die Temperatur um 50 °C schwanken kann. Besonders bevorzugt erfolgt die Zersetzung und Abscheidung in einem Hotwall-Reaktor.

Die Bildung der Siliciumpulver kann erfolgen durch 1) Zersetzen der Siliciumverbindung, 2) Abkühlen des gasförmigen Siliciums, insbesondere in Gegenwart mindestens eines inerten Verdünnungsgases und/oder 3) die Siliciumpulver gebildet werden durch ein Inkontaktbringen der zersetzten Siliciumverbindung mit einem kühleren reaktiven Gas unter Bildung und Abkühlen der des eines amorphen Siliciumpulvers umfassend eine Siliciumlegierung und Abscheidung des Pulvers.

Ebenso Gegenstand der Erfindung ist ein Verfahren insbesondere umfassend die folgende Verfahrensführung: (A) Zersetzung der Siliciumverbindung in Gegenwart eines Verdünnungsgases und optional mindestens eines reaktiven Gases unter (i) thermischen Bedingungen und/oder (ii) im Plasma, und Ausbildung von Primärpartikeln und/oder Clustern der Primärpartikel, optional umfassend eine Siliciumlegierung, mit den Schritten - (B) Bildung von amorphen Primärpartikeln umfassend mindestens eine intrinsische Zone einer Siliciumlegierung, und/oder Clustern der Primärpartikel und/oder- (C) Bildung von reinen, amorphen Siliciumprimärpartikeln und/oder Clustern, wobei die in (B) oder (C) gebildeten Primärpartikel und/oder Cluster unter (i) thermischen Bedingungen und/oder in (ii) einer Behandlung im Plasma erfolgt und optional - (D.1) die Partikel aus (B) und (C) unter (i) thermischen Bedingungen und/oder in (ii) einer Behandlung im Plasma mit mindestens einer Zone einer Siliciumlegierung zumindest teilweise beschichtet werden.

Ferner ist Gegenstand der Erfindung ein Verfahren umfassend vorzugsweise die Schritte (i) thermische Zersetzung zumindest teilweise an einer heißen Innenwand in einem Reaktionsraum und/oder (ii) im Plasma in einem Reaktionsraum in einem Reaktor, insbesondere einem Freiraum-Infrarotstrahlungs-Reaktor und oder Gasentladungsreaktor, umfassend einen Reaktionszylinder, in dem im Bereich des Reaktoranfangs in den Reaktionsraum eines Reaktionszylinders über mindestens eine Zuführung die Siliciumverbindung, optional das Verdünnungsgas, insbesondere optional als Gemisch umfassend das reaktive Gas und/oder das Verdünnungsgas, in den Reaktionsraum eingebracht wird, der Reaktionsraum umfasst mindestens eine Reaktionszone, vorzugsweise umfasst der Reaktionsraum eine Vielzahl an definierten Reaktionszonen, in denen die Temperatur definiert einstellbar ist. In mindestens einer Reaktionszone erfolgt die Zersetzung der Siliciumverbindung und optional die Bildung der Siliciumpulver, und optional erfolgt eine Legierung und/oder Beschichtung der gebildeten Siliciumpulver, insbesondere mit einer Bor enthaltenden Schicht. Am Reaktorausgang werden die gebildeten Primärpartikel und/oder Cluster erhalten, insbesondere aus dem Reaktor ausgeschleust.

Die Temperaturbestimmung zur Kalibrierung der Reaktor-Thermofühler erfolgt bevorzugt mit der dem Fachmann bekannte Glühdraht-Vergleichsmessung.

Erfindungsgemäß und bevorzugt werden mittels Gasentladung nichtthermische Plasmen erzeugt und finden im Verfahren Anwendung.

Erfindungsgemäß eingesetzte nicht-thermische Plasmen werden beispielsweise durch eine Gasentladung oder durch Einstrahlung von elektromagnetischer Energie, wie durch Einstrahlung von Radio- oder Mikrowellen, in einer Unterdruckkammer erzeugt. Die Erzeugung des Plasmas erfolgt also nicht wie bei thermischen Plasmen durch hohe Temperaturen, sondern durch nichtthermische Ionisationsprozesse. Dem Fachmann sind solche Plasmen bekannt.

Für die vorstehend aufgeführten Verfahren wurden im nichtthermischen Gleichgewicht betriebene Gasentladungen verwendet. Nichtthermisch bedeutet im erfinderischen Sinne, dass die Elektronen als energievermittelnde Spezies eine höhere Temperatur und/oder eine höhere Energie als die Schwerteilchen (Xe, Ar, XeAr, Xe₂, N₂, N₂⁺, Kr, Si, SiH, SiH₂⁺ ... C, H, H₂, N, NH, ...) aufweisen. Diese wurden durch dem Fachmann bekannte bzw. geläufige Netzteile erzeugt. Das nichtthermische Plasma weist im Allgemeinen Elektronen mit einer Energie im Bereich von 0,1 bis 100 eV, insbesondere von 1 bis 50 eV, auf und Schwerteilchen mit einer Energie im Bereich von 1 bis 10 eV, insbesondere 0,5 bis 1 eV. Elektrodenabstand 4 mm. Die Teilchenenergie kann bspw. mittels Laserspektroskopie bestimmt werden. (Verfahrensanweisung zur Bestimmung können bei den jeweiligen nationalen Eich-Anstalten angefordert werden oder sind den ISO-Normen und/oder anderen Fachstandards zu entnehmen).

Überraschenderweise hat sich gezeigt, dass sich eine nicht-thermische Gasentladung vorteilhaft durch Dimmen über die Pulsfrequenz erzeugen lässt, wobei vorteilhafterweise die Elektroden als Tubularektroden mit vorzugsweise porösen Wandflächen bspw. aus Sintermetall durch eine zweidimensionale zirkular-parabolische Innen-Form ausgeführt sind. Damit verteilt sich der Gasstrom schraubenförmig vorwärts, beabstandet von der Elektrodenoberfläche.

Das erfindungsgemäße Verfahren wird im Allgemeinen in nicht-thermischen Plasmazuständen durchgeführt, welche Schwerteilchen-Temperaturen von 50 bis 1400 °C, vorzugsweise von 100 bis 1000 °C aufweisen.

Das Plasma kann gepulst werden, vorzugsweise wird ein im Wesentlichen zylinderförmiges Plasma, insbesondere nicht-thermisches Plasma, in einem zylindrischen Bereich des Reaktionszylinders vorgesehen, in dem eine vorteilhaft homogene Dotierung und/oder Zone einer Siliciumlegierung mit Zersetzungsprodukten aus einem reaktiven Gas oder Gemisch von Gasen, der oberhalb im Reaktionszylinder gebildeten partikelförmigen siliciumhaltigen Partikel erfolgt.

Eine weitere bevorzugte Ausführungsform des erfindungsgemäßen Verfahrens beinhaltet das Einleiten von Edelgas oder von Edelgasgemischen als Verdünnungsgas in das nicht-thermische Plasma, das insbesondere Temperaturen von kleiner gleich 1500 °C, vorzugsweise kleiner gleich 1300 °C, bevorzugt kleiner gleich 1100 °C aufweist.

Ein besonderer Vorteil des erfindungsgemäßen Verfahrens ist die effiziente Abtrennung der Partikel von gasförmigen Komponenten, wie dem Verdünnungsgas oder nicht umgesetzten reaktiven Gasen. So erfolgt vorzugsweise die Abtrennung der Partikel, indem diese durch eine gasdurchlässige Membran aufgefangen werden. So können die Partikel optional kontinuierlich oder diskontinuierlich über eine abschüssig angeordnete Membran abgetrennt werden. Das Verdünnungsgas kann mithilfe der Membran abgetrennt, aufgearbeitet und erneut verwendet werden.

In dem Verfahren nach der Erfindung werden vorzugsweise bei erhöhter Temperatur gasförmige siliciumhaltige Verbindungen umgesetzt, die umfassen Wasserstoff- und/oder Halogen-enthaltende Silane, wie H-Silane, Halogensilane, und/oder Kohlenwasserstoff-enthaltende Silane, wie Methylsilan, Methyltrichlorsilan, Dimethyldichlorsilan, Halogensilane, bevorzugt auch Polyperchlorsilangemische umfassend Polyperchlorsilane mit 2 bis 8 Siliciumatomen besonders bevorzugt ist auch die Verwendung höhermolekularer Polychlorsilane mit mindestens drei Siliciumatomen, insbesondere mit 3 bis 8 Siliciumatomen. Zur Bestimmung der Anteile mit den vorgenannten Elementen und Verbindungen erfolgt vorzugsweise die Analyse mittels GC und/oder ICP-MS.

Dabei ist es bevorzugt, dass die Primärpartikel und/oder Cluster der Primärpartikel in einer Vorrichtung zur Partikelabscheidung aufgefangen werden, insbesondere mittels einer Gaswäsche oder Filtration bspw. einem Sackfilter und/oder gasdurchlässiger Membranen oder alternativ mit einem Filterschlauch.

Verfahrensgemäß kann die Legierung der Primärpartikel und optional der Primärpartikel der Cluster erfolgen mit a) Elementen der dritten Hauptgruppe des Periodensystems der Elemente (Gruppe 13 des Periodensystems der Elemente nach IUPAC), wie Bor, Aluminium, und/oder Elementen der fünften Hauptgruppe (Gruppe 15 des Periodensystems der Elemente nach IUPAC) wie Stickstoff, Phosphor, Arsen, Antimon, bevorzugt kann eine Legierung durch Zusatz von Diboran erfolgen, und/oder es kann eine Modifizierung der Primärpartikel und optional der Cluster erfolgen b) zumindest teilweise mit mindestens einem organofunktionellen Silan.

Besonders bevorzugte Abkühlungsbedingungen werden nachfolgend näher erläutert. Wie vorstehend ausgeführt kann die Bildung der Pulver auf zweierleiweise erfolgen, so kann die Abkühlung und Ausbildung der Siliciumpulver durch Reaktion mit kühlen reaktiven Gasen, insbesondere flüssigem Stickstoff erfolgen ggf. mit einem Gehalt an Kohlenwasserstoffen, Bor-, Phosphor-Verbindungen und/oder Verbindungen der Elemente der dritten Hauptgruppe des Periodensystems der Elemente (Gruppe 13 des Periodensystems der Elemente nach IUPAC), wie Bor, Aluminium, und/oder Elemente der fünften Hauptgruppe (Gruppe 15 des Periodensystems der Elemente nach IUPAC), wie Stickstoff, Phosphor, Arsen, Antimon.

Die Abkühlung der Siliciumpulver kann auch erfolgen durch Einleiten des gasförmigen Siliciums in flüssiges Kühlmittel, wie flüssiges Helium oder in ein flüssiges reaktives Gas. In diesem Fall bilden sich die beschichten Siliciumpartikel unmittelbar in dem reaktiven Gas. Nach einer alternativen Verfahrensvariante erfolgt die Abkühlung des Siliciums in einer kühleren Region des Reaktors, die vorzugsweise eine Temperatur im Bereich kleiner gleich 1000 °C aufweist. Eine Abkühlung kann auch durch ein Einleiten in inerte, kühle und leicht verdampfbare Flüssigkeiten oder durch Einleiten in flüssige Siliciumverbindungen oder Bor enthaltende erfolgen. Generell kann das gasförmige Silicium abgekühlt werden, indem es in eine Zone des Reaktors oder Reaktionszone geleitet wird, dessen Temperatur deutlich unterhalb 1000 °C liegt, vorzugsweise unter 900 °C, weiter bevorzugt unterhalb 800 °C, besonders bevorzugt unterhalb 600 °C. Besonders bevorzugt erfolgt ein rasches Abkühlen durch Einstellen einer Temperaturdifferenz von größer gleich 50 °C, insbesondere größer gleich 100 °C, bevorzugt von größer gleich 200 °C, besonders bevorzugt größer gleich 500 °C unterhalb des Schmelzpunktes von Silicium oder des Schmelzpunktes der Beschichtung., innerhalb von kleiner gleich 100 Millisekunden, um im Wesentlichen amorphe Siliciumpulver zu erhalten.

Um im Wesentlichen amorphe Siliciumpulver zu erhalten erfolgt vorzugsweise eine rasche Abkühlung indem innerhalb sehr kurzer Zeit eine starke Temperaturerniedrigung erfolgt. So kann die Zone zur Zersetzung und/oder Legierung eine Temperatur von 400 bis 1500 °C aufweisen, während die Abscheidung und optional Beschichtung bei -kleiner gleich 600 °C, vorzugsweise unter 200 °C bis hin zu -170°C oder bei Stickstoff-Siede-Temperatur erfolgt.

Besonders effizient zur Herstellung amorpher Partikel ist eine Verfahrensführung analog der Sprühtrocknung, in dem mit einem starken Druckabfall von 1 bar auf 200 mbar(abs), der in den gasförmigen Zustand überführten Siliciumverbindung und optional in Gegenwart eines Verdünnungsgases gearbeitet wird. Alternativ kann das reaktive Gas nach dem starken Druckabfall mit den gebildeten Primärpartikeln des Siliciums in Kontakt gebracht werden, um die Beschichtung auf die Partikel und optional Cluster aufzubringen.

Alternativ können die amorphen Primärpartikel erhalten werden in einem nicht-thermische Plasma, erzeugt mittels Dielektrisch Behinderter Entladung wie sie für einen Ozonisator mit Schlagweiten von mindestens 1 mm typisch ist, deren Schwerteilchen-Temperaturen bei kleiner gleich 900 °C, vorzugsweise kleiner gleich200 °C bis hin zu weniger als Minus 100 °C (entsprechend weniger als 173°Kelvin) liegen.

Erfindungsgemäß bevorzugt umfasst das Plasma die Bedingungen einer Gasentladung, insbesondere nicht-thermischer Gleichgewichtsbedingungen im Plasma. Erfindungsgemäß eingesetzte nicht-thermische Plasmen werden beispielsweise durch eine Gasentladung oder durch Einstrahlung von elektromagnetischer Energie, wie durch Einstrahlung von Radio- oder Mikrowellen, in einer Druckkammer erzeugt. Die Erzeugung des Plasmas erfolgt also nicht wie bei thermischen Plasmen durch hohe Temperaturen, sondern durch nicht-thermische Ionisationsprozesse. Dem Fachmann sind solche Plasmen bekannt.

Das Plasma kann gepulst werden, vorzugsweise wird ein im Wesentlichen reaktives Plasma, insbesondere nicht-thermisches Plasma, in einem zylindrischen Bereich des Reaktionszylinders/- raumes vorgesehen, in dem eine vorteilhaft homogene Legierung und/oder Beschichtung mit Zersetzungsprodukten aus einem reaktiven Gas oder Gemisch von Gasen, der oberhalb im Reaktionszylinder gebildeten partikelförmigen Siliciumpartikel erfolgt.

Eine weitere bevorzugte Ausführungsform des erfindungsgemäßen Verfahrens beinhaltet das Einleiten von Edelgas oder von Edelgasgemischen als Verdünnungsgas in das nicht-thermische Plasma, das insbesondere Temperaturen von kleiner gleich 1500 °C, vorzugsweise kleiner gleich 1300 °C, bevorzugt kleiner gleich 1100 °C aufweist.

Ein besonderer Vorteil des erfindungsgemäßen Verfahrens ist die effiziente Abtrennung der Partikel von gasförmigen Komponenten, wie dem Verdünnungsgas oder nicht umgesetzten reaktiven Gasen. So erfolgt vorzugsweise die Abtrennung der Partikel, indem diese durch eine gasdurchlässige Membran aufgefangen werden. So können die Partikel optional kontinuierlich oder diskontinuierlich über eine abschüssig angeordnete Membran abgetrennt werden. Das Verdünnungsgas kann mithilfe der Membran abgetrennt und erneut verwendet werden.

In dem Verfahren nach der Erfindung werden vorzugsweise bei erhöhter Temperatur gasförmige Siliciumverbindungen umgesetzt, die umfassen Wasserstoff- und/oder Halogen-enthaltende Silane, wie H-Silane, Halogensilane, und/oder Kohlenwasserstoff-enthaltende Silane, wie Methylsilan, Methyltrichlorsilan, Dimethyldichlorsilan, Halogensilane, Chlorsilane sowie reine, hochreine, insbesondere höchstreine H-Silane, wie Monosilan und/oder Chlorsilane, wie Dichlorsilan, Trichlorsilan, Tetrachlorsilan, Hexachlordisilan, Methyltrichlorsilan, Polyhalogensilane, Wasserstoff enthaltende Polysilane, umfassend ausschließlich Wasserstoff oder Wasserstoff und Halogen. Zur Herstellung der amorphen Siliciumpulver können gleichfalls Alkoxsilane, vorzugsweise Tetramethoxysilan, Tetraethoxysilan oder gemischte Tetraalkoxysilane eingesetzt werden.

Vorzugsweise beträgt der Gehalt an Silanen, insbesondere an monomeren und/oder Polysilanen, bevorzug an Polychlorsilanen und/oder Polyperchlorsilangemische größer gleich 98 Gew.-% bis 99,9999999 Gew.-%, mit kleiner gleich 100 Gew.-% bis 0,001 Gew.-ppt Gesamtverunreinigungen in einem hochreinen Silan, vorzugsweise kleiner 50 Gew.-ppm bis 0,001 Gew.-ppt in einem höchstreinen Silan, und ggf. ad 100 Gew.-% Polysilane.

Erfindungsgemäß einsetzbare Silane umfassen Silane der allgemeinen Formel I,

HₓSiCl₄₋ₓ (I)

mit x unabhängig voneinander ausgewählt aus 0, 1, 2 oder 3, vorzugsweise ist x gleich 0, 1 oder 2, bevorzugt ist x gleich 0 oder 1, besonders bevorzugt ist x gleich 0, oder ein Gemisch umfassend mindestens zwei monomere Chlorsilane der Formel I, insbesondere ausgewählt aus Tetrachlorsilan, Trichlorsilan und Dichlorsilan, bevorzugt reines Tetrachlorsilan oder reines Tetrachlorsilan mit einem Gehalt an Trichlorsilan und/oder Dichlorsilan.

Bevorzugt können auch Polyperchlorsilangemische umfassend Polyperchlorsilane mit 2 bis 8 Siliciumatomen eingesetzt werden. Besonders bevorzugt ist auch die Herstellung höhermolekularer Polychlorsilane mit mindestens drei Siliciumatomen, insbesondere mit 3 bis 8 Siliciumatomen. Zur Bestimmung der Gesamtverunreinigung mit den vorgenannten Elementen erfolgt vorzugsweise mittels ICP-MS.

Bevorzugt sind Polyperchlorsilangemische umfassend Polyperchlorsilane mit 2 bis 8 Siliciumatomen. Besonders bevorzugt ist auch die Herstellung höhermolekularer Polychlorsilane mit mindestens drei Siliciumatomen, insbesondere mit 3 bis 8 Siliciumatomen.

Polychlorsilane entsprechend der Erfindung umfassen die homologe Reihe der Polyperchlorsilane der allgemeinen Formel II SiₙCl₂ₙ₊₂, mit n grösser gleich 2, welche lineare und/oder verzweigte Ketten bilden sowie die Polyperchlorsilane, die Ringe oder Polymere bilden, wobei die Polymere auch verzweigt und/oder cyclisch sein können, mit der idealisierten Formel III SiₙCl₂ₙ , mit n grösser gleich 3, als auch die Siliciumchloride mit geringerem Chlorgehalt der idealisierten Formel IV SiCl_{1,5}.

Besonders bevorzugt gelten als Polychlorsilane Verbindungen der allgemeinen Formel II Si'Cl₂ₙ₊₂, mit n grösser gleich 2, insbesondere mit n grösser gleich 2 bis 100, vorzugsweise mit n grösser gleich 2 bis 50, vorzugsweise jeweils unabhängig mit n grösser gleich 2, 3, 4, 5, 6, 7, 8, 9 oder 10, bevorzugt 2 bis 8, besonders bevorzugt mit n gleich 2 oder 3, wobei sie lineare als auch verzweigte Ketten bilden können; und Verbindungen der allgemeinen Formel III, die Ringe oder Polymere bilden mit Si'Cl₂ₙ, mit n grösser gleich 3, insbesondere mit n grösser gleich 4 bis 100, insbesondere mit n grösser gleich 4 bis 50, besonders bevorzugt jeweils unabhängig mit n grösser gleich 4, 5, 6, 7, 8, 9 oder 10, als auch Polychlorsilane mit geringerem Chlorgehalt gemäss der allgemeinen Formel IV mit Si'Cl_{1,5,n}, mit n grösser gleich 4 oder 5, insbesondere mit n grösser gleich 6 bis 200, vorzugsweise mit n grösser gleich 8 bis 100.

Besonders bevorzugt wird ein Polychlorsilan (PCS) eingesetzt, insbesondere Octachlortrisilan oder ein Octachlortrisilan in einer Mischung mit höhermolekularen Polychlorsilanen, vorzugsweise Polyperchlorsilanen, wobei das Polychlorsilan insbesondere einen Gehalt an Octachlortrisilan von 20 bis 99,9999 Gew.-% aufweist, vorteilhaft mit einem vorgenannten Verunreinigungsprofil.

Es kann auch ein gelöstes Polysilan und/oder Polychlorsilan beispielsweise in einem reaktiven Lösemittel, wie Kohlenwasserstoff als Flüssigkeit, Sirup, Paste, Creme, Dispersion, Emulsion, eingesetzt werden, wobei das hochreine Lösemittel vor der Zersetzung zu einem reaktiven Gas verdampft wird. Somit gelten auch reaktive verdampfbare Lösemittel als reaktive Gase.

Vorzugsweise wird in dem Verfahren ein inertes Verdünnungsgas verwendet. Je nach Anforderungsprofil kann auf das Verdünnungsgas verzichtet werden, wenn zugleich ein Vakuum anliegt.

Bevorzugte Verdünnungsgase sind Argon, Helium, Xenon, Krypton oder ein Gemisch umfassend mindestens zwei der genannten Gase. Die Zugabe von Edelgasen, vorzugsweise von Argon und Xenon im Verfahren begünstigen die Bildung sphärischer Partikel. In dem erfindungsgemäßen Verfahren werden die gasförmige Siliciumverbindung die Verdünnungsgase und optional reaktives Gas als Gesamtzusammensetzung von 100 Vol.-% in dem Verfahren eingesetzt. Dabei ist es besonders bevorzugt, wenn die Verdünnungsgase mindestens zu 0,0001 bis 99 Vol.-%, insbesondere von 0,0001 bis 60 Vol.-%, bevorzugt von 0,1 bis 1,0 Vol.-% Edelgase enthält, insbesondere Xenon und/oder Argon. Somit ist es bevorzugt von 0,0001 bis 99 Vol.-%, insbesondere von 0,0001 bis 60 Vol.-%, bevorzugt von 0,1 bis 1,0 Vol.-% Edelgase in dem Verfahren einzusetzen, insbesondere Xenon und/oder Argon.

Stickstoff ist unter den genannten Reaktionsbedingungen kein inertes Verdünnungsgas, sondern ein reaktives Gas. Das Verdünnungsgas kann vorzugsweise mit der gasförmigen Silicium-Verbindung und/oder dem reaktiven Gas vor dem Einbringen in den Reaktionszylinder gemischt werden. Alternativ wird das inerte Verdünnungsgas zur Abkühlung der Zersetzungsprodukte, wie beispielsweise zur Abscheidung der Pulver zugegeben. Die Zersetzung der Silicium-Verbindung und optional des reaktiven Gases kann auch durch Überführen der in einen Hochvakuum-Bereich erfolgen. Als ein Hoch-Vakuum wird ein Vakuum kleiner gleich 0,01 bar, insbesondere kleiner gleich 0,001 bar, kleiner gleich 0,0001 bar erachtet.

Der Druckbereich liegt üblicherweise bei 1 mbar bis 50 bar, insbesondere bei 1 mbar bis 10 bar, bevorzugt bei 1 mbar bis 5 bar. Je nach dem gewünschten Zersetzungs- und/oder Legierungs- und/oder Beschichtungsprodukt und, um die Bildung von Kohlenstoff enthaltenden Prozessgasen zu minimieren, kann das Verfahren auch in einem Druckbereich von 1 bis 50 bar erfolgen, bevorzugt bei 2 bis 50 bar, besonders bevorzugt bei 5 bis 50 bar. Dabei weiß der Fachmann, dass der zu wählende Druck ein Kompromiss zwischen Gasabführung, Agglomerierung und Reduktion der Kohlenstoff enthaltenden Prozessgase ist.

Bevorzugt einsetzbare reaktive Gase als Legierungs- oder Beschichtungsverbindung umfassen Stickstoff enthaltende Verbindungen, Germanium enthaltende Verbindungen, Antimonhaltige Verbindungen, Wasserstoff, Stickstoff (N₂), NH₃, Hydrazin, Stickstoffwasserstoffsäure, Kohlenwasserstoffe, wie Methan, Butan, Propan, höchstreines Erdgas, aromatische Verbindungen, wie Toluol, insbesondere jeweils sauerstofffreie Verbindungen, mit der Maßgabe, dass sich bei der Zersetzung kein Wasser bildet. Bevorzugte reaktive Gase umfassen Kohlenwasserstoffe, wie Methan, Ethan, Propan, Butan, Gemische dieser etc., HCl, Kohlenwasserstoffe mit Stickstoff. Zur Bildung der Bor enthaltenden Beschichtungen wird bevorzugt Diboran, Bortrichlorid oder einer andere leicht zersetzbare Borverbindung eingesetzt. Gleichfalls besonders bevorzugte reaktive Gase umfassen Verbindungen der vorgenannten Elemente (reaktive Gase) der dritten Hauptgruppe des Periodensystems der Elemente (Gruppe 13 des Periodensystems der Elemente nach IUPAC), wie Aluminium, und/oder Elementen der fünften Hauptgruppe (Gruppe 15 des Periodensystems der Elemente nach IUPAC ), wie Phosphor, Arsen, Antimon.

Das reaktive Gas kann auch in einer Plasmareaktionszone mit einer Dotierverbindung wie Diboran oder Borhalogenid, insbesondere Diboran in einem Plasma umgesetzt werden. Je nach Verfahrensführung werden die Dotierverbindungen bevorzugt zur Ausbildung einer zumindest teilweisen Beschichtung der Primärpartikel und/oder der Cluster eingesetzt werden. Bevorzugt ist eine vollständige Beschichtung der Partikel und optional der Cluster.

Als reaktive Gase gelten auch mit Bor, Aluminium, Antimon, Selen, und/oder Phosphor angereicherte Gase oder Gasgemische, die in Aufreinigungsprozessen zur Herstellung höchstreiner Verbindungen als Nebenstrom bzw. Abfallprodukte erhalten werden. Diese reaktiven Gase können bei konstanten Gehalten an Bor, Aluminium, Phosphor etc., die hier als Legierungskomponente verwendet werden, zur Herstellung der legierten und/oder beschichteten Siliciumpulver eingesetzt werden.

Die Beschichtung kann im (vorzugsweise gepulsten) Gleichstrom(niederdruck)plasma durch chemische Gasphasenabscheidung erfolgen. Generell kann die Legierung in einem Verfahren im Gleichstrom oder auch im Gegenstrom (Gasstrom) oder in gekreuzten Gasströmen erfolgen. Eine definierte Legierung oder Beschichtung kann durch seitliche Zufuhr eines reaktiven Gases oder eine Zufuhr im Gegenstrom gut homogenisiert und gesteuert werden.

Generell kann die thermische Behandlung die Zersetzung umfassen sowie eine thermische Nachbehandlung, die zunächst ein starkes Abkühlen auf vorzugweise kleiner gleich 800 °C zur Ausbildung der amorphen Partikel umfasst, die anschließend in einem weiteren Verfahrensschritt zumindest teilweise in einem Plasma oder in einer thermischen Behandlung beschichtet werden können. Zusätzlich oder alternativ kann durch eine thermische Nachbehandlung auch eine Ausbildung der Cluster erfolgen und deren Geometrie gesteuert werden.

Die Siliciumverbindungen können mittels mindestens einer Düse, Vernebler oder ähnliches in die Gasphase überführt werden oder als gasförmige Siliciumhaltige Verbindung optional als Gemisch umfassend reaktive Gase und/oder das inerte Verdünnungsgas in den Reaktionsraum eingebracht werden.

Zur Durchführung des Verfahrens ist es zusätzlich zu den vorgenannten Merkmalen weiter bevorzugt, wenn die Gasentladung ein nicht-thermisches Plasma ist, weiter bevorzugt erfolgt die Gasentladung in einer einem Ozonisator nachempfunden Reaktor mit größerer Schlagweite (GAP, Spaltweite zwischen den Elektroden). Für die physikalische Definition des nichtthermischen Plasmas und der homogenen Plasmakatalyse wird auf die einschlägige Fachliteratur verwiesen, wie beispielsweise auf "Plasmatechnik: Grundlagen und Anwendungen - Eine Einführung; Autorenkollektiv, Carl Hanser Verlag, München/Wien; 1984, ISBN 3 446-13627-4".

Besonders bevorzugt beträgt der spezifische auf das Reaktorvolumen bezogene Energieeintrag von 0,1 und 1000 Ws/cm³. Dabei ist es weiter bevorzugt, wenn der spezifische Energieeintrag mittels phasengenauer Momentanleistungsmessung mit einer Bandbreite von mindestens 250 kHz durchgeführt wird, wobei die Bestimmung der Momentanleistungsmessung in einem koaxialen Reaktor mit 50 cm² Entladungsfläche erfolgt. Ein koaxialer Reaktor ist vorzugsweise ein Rohrreaktor, insbesondere rotationssymmetrischer Rohrreaktor.

Der Energieeintrag zur Ausbildung des nicht-thermischen Plasmas erfolgt vorzugsweise in der Art, dass sich in dem sich ausbildenden Plasma möglichst homogene Bedingungen für die Umsetzung der Silane mit C, N und/oder Ge enthaltenen Verbindungen ausbilden, dabei ist es besonders bevorzugt, wenn das nicht-thermische Plasma bei einer Spannung betrieben wird, bei der die Entladung die gesamte Elektrodenfläche bedeckt (normale Glimmentladung).

Entsprechend einer bevorzugten Alternative werden die abgeschiedenen Partikel an ggf. oberflächlich vorhandenen Chlor-Atomen organofunktionalisiert. Vorzugsweise werden die Partikel an der Oberfläche der Partikel durch Reaktion mit einer reaktiven organofunktionellen Gruppe des Silans modifiziert. Die Modifizierung kann über Komplexierung oder eine Ausbildung kovalenter Bindungen erfolgen. Generell können die Partikel der Siliciumpulver zumindest teilweise mit einem organofunktionellen Silan modifiziert werden.

Organofunktionelle Silane umfassen Silane mit ungesättigten Kohlenwasserstoffresten, Halogenfunktionalisierte Silane, wie vorzugsweise Halogenalkylsilane, wie Monochlortrimethylsilan, Halogenalkoxysilane, wie Monochlortrialkoxysilan, Alkylenalkoxysilane, Alkylenhalogensilane, Amino-funktionelle Silane, wie Aminopropyltriethoxysilan, Aminopropyltrialkylsilan, sowie organofunktionelle Silane. Organofunktionelle Silane umfassen auch organisch funktionalisierte Siliciumverbindungen und Organosiloxanen.

Gegenstand der Erfindung sind auch Formulierungen enthaltend die Siliciumpulver umfassend optional Hilfsmittel und Lösungsmittel und/oder Dispersionsmittel und/oder Suspensionsmittel.

Gegenstand der Erfindung ist auch ein Reaktor (Freiraum-Infrarotstrahlungs- Reaktor) **1** zur Herstellung von Siliciumpulvern mit Partikeln deren Primärpartikelgröße von 1 nm bis 100 nm liegt, wobei der Reaktor **1** einen Reaktionszylinder **2** mit Reaktionsraum **2.1** umfasst und im Bereich des Reaktoranfangs **2a, 3** mindestens zwei Zuführungen **4.1, 4.2, 4.3** aufweist für die Einleitung a) der mindestens einen bei erhöhter Temperatur gasförmigen Siliciumverbindung, und b) optional des mindestens eines reaktiven Gases und/oder Verdünnungsgases, in den Reaktionsraum **2.1,** wobei dem Reaktionsraum **2.1** zumindest in einem Bereich am äußeren Umfang des Reaktionsraumes **2.1** Heizvorrichtungen **7** zugeordnet sind, und/oder dem Reaktionsraum **2.1** mindestens ein Plasmagenerator **9** zugeordnet ist, zur Erzeugung eines Plasmas im Reaktionsraum **2.1,** und wobei dem Reaktor **1** mindestens eine, vorzugsweise zwei Zuführungen **4, 4.1, 4.2** und/oder **4.3** im Bereich des Reaktoranfangs **2a** oder **3** zugeordnet sind. Nach der Alternative mit zwei Zuführungen sind die Zuführungen bevorzugt in einem Winkel von größer gleich 30° zueinander angeordnet. Im Reaktionsraum **2.1** ist mindestens eine Reaktionszone **2.1a,** alternativ sind mehrere Reaktionszonen **2.1a** bis **2.1n** im Reaktionsraum angeordnet. Vorteil der verschiedenen Reaktionszonen ist die Möglichkeit eine definierte Temperaturführung über die Reaktorläge einzustellen.

Vorzugsweise weist die Zuführung **4** einen Winkel von 0 bis 45° zur Mittelachse des Reaktionszylinders **2** auf, vorzugsweise 0 bis 10° und die optionale zweite Zuführung **4.3** einen Winkel von größer 45 bis 135° zur Mittelachse des Reaktionszylinders **2,** insbesondere um 80 bis 100°, bevorzugt um 90°. Die zweite Zuführung kann auch zur Einstellung der Strömung der gasförmigen Siliciumverbindung mit einem Verdünnungsgas optional im Gemisch mit einer Siliciumverbindung zudosiert werden.

Optional kann der Reaktor zwischen dem Reaktoreingang und Reaktorausgang über weitere Zuführungen verfügen, die für die Legierung und/oder Beschichtung der Siliciumpulver verwendet werden können. So kann beispielsweise über eine Zuführung im mittleren Bereich des Reaktorzylinders eine Beschichtung der Partikel erfolgen, indem beispielswiese eine Bor enthaltenden Verbindung in den Reaktionszylinder, insbesondere in eine Reaktionszone des Reaktionsraumes zugeführt wird.

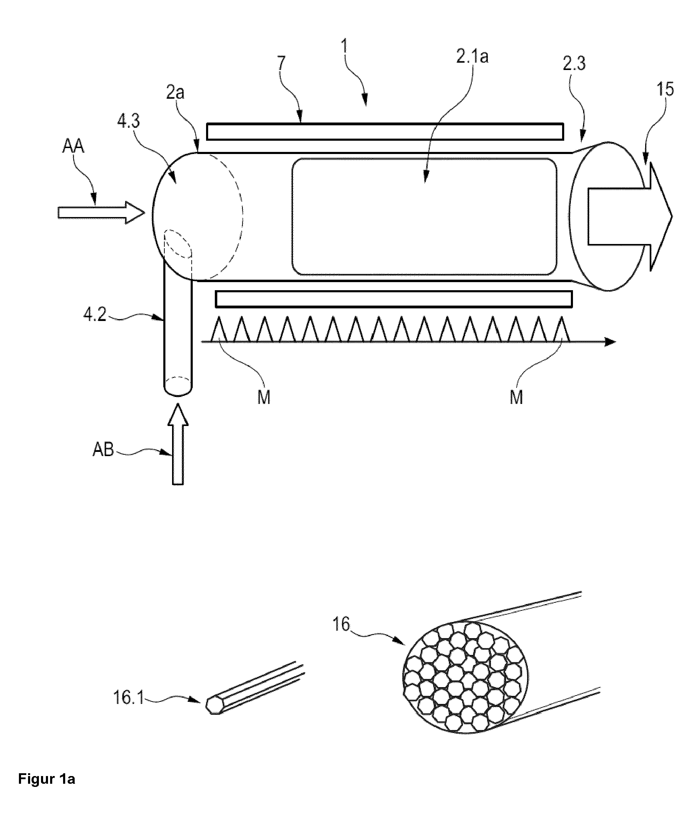

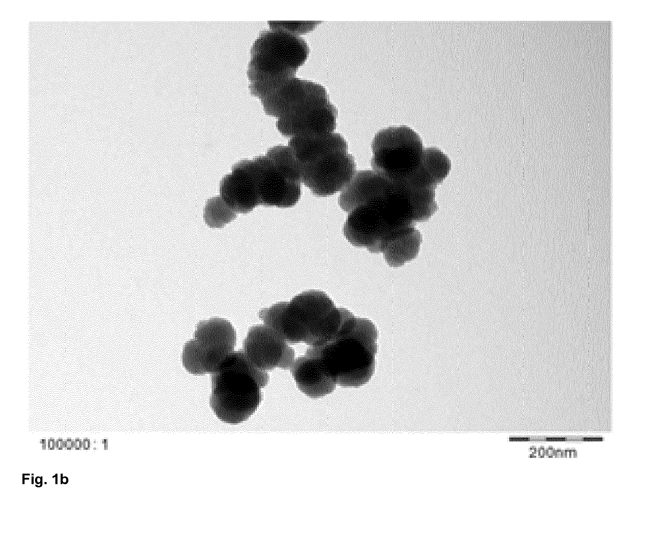

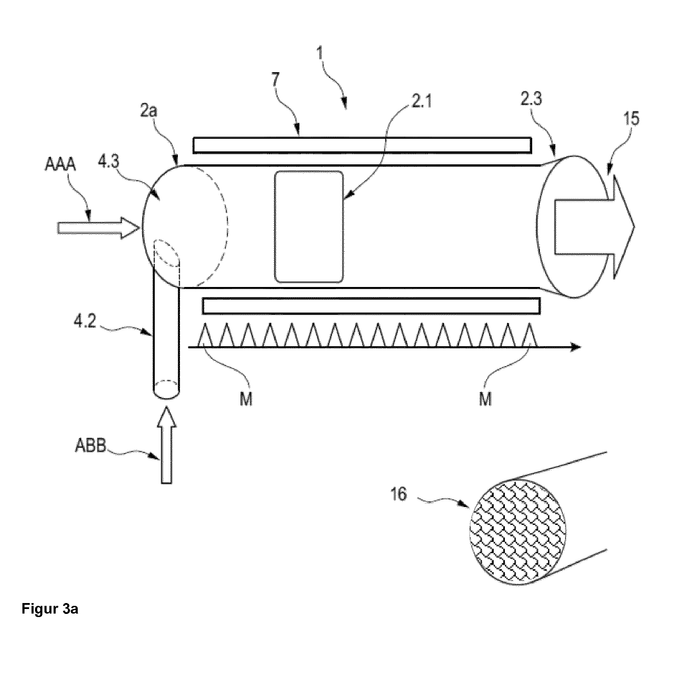

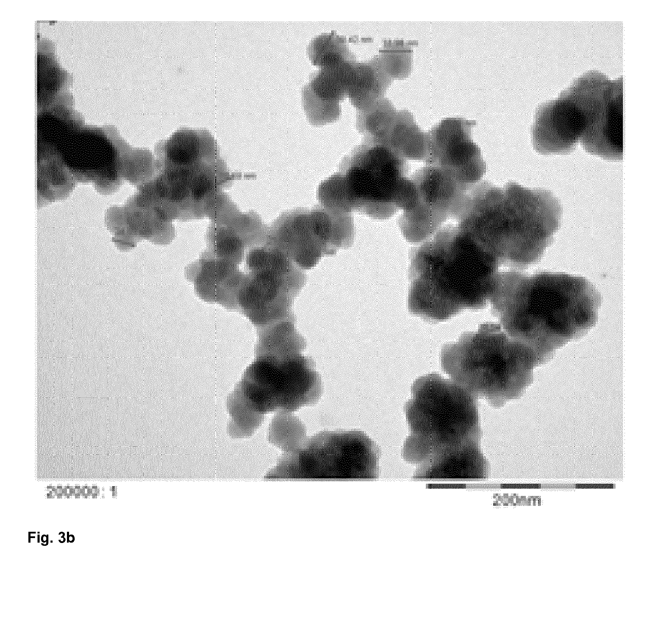

Der Reaktionsraum und oder die mindestens eine Reaktionszone kann zudem gemäß **Figur 1** und **3** einen Reaktoreinsatz mit Milli-Reaktionskanälen aufweisen. Der Reaktoreinsatz mit Millireaktionskanälen kann aus Siliciumcarbid gefertigt sein oder aus jedem anderen geeigneten Material. Bevorzugt sind SiC, Siliciumnitrid und/oder Graphit. Der Reaktoreinsatz dient dazu eine sehr schnelle Zersetzung und sehr schnelle Abscheidung über die Einstellung von Temperaturdifferenzen über sehr kurze Wegstrecken im Reaktionsraum und damit in den einzelnen Reaktionszonen zu gewährleisten.

Nach einer alternativen bevorzugte Ausführungsform weist der Reaktionsraum **2,** insbesondere in mindestens einer Reaktionszone **2.1a,** eine Reaktoreinsatz **16** mit Kanälen auf, wobei der Reaktoreinsatz vorzugsweise innere Kanälen umfasst, deren Durchmesser 1 bis 20 mm betragen, vorzugsweise 1 bis 10 mm, vorteilhaft sind die Kanäle rohrförmig, wobei ein rohrförmiger Kanal im Querschnitt jede beliebige Geometrie umfassend kreisförmig, wabenförmig etc. aufweisen kann.

Vorteilhaft verfügt der Reaktionsraum **2.1** über mindestens eine Reaktionszone **2.1a** bis **2.1n,** insbesondere über 2 bis 100 Reaktionszonen. In jeder Reaktionszone können definierte thermische Bedingungen oder Plasmabedingungen eingestellt werden.

Der Reaktor verfügt vorzugsweise im Bereich des Reaktorausgangs **2.2, 2.3** über eine Vorrichtung für die Auftrennung von Reaktionsprodukten, insbesondere für die Abtrennung des festen partikelförmigen Siliciumpulvers, vorzugsweise eine Vorrichtung zur Partikelabscheidung **15,** bevorzugt eine Vorrichtung umfassend eine gasdurchlässige Membran **10.**

Der Reaktionsraum ist evakuierbar, so dass ein definiertes Vakuum einstellbar ist.

Das Plasma in der Entladungszone kann durch Hochspannungspulse in einfacher Weise als Plasma im nichtthermischen Gleichgewicht erzeugt werden. Die Temperaturen der Schwerteilchen (Atome, Ionen) sowie der Leichtteilchen (Elektronen) richtet sich nach reaktionstechnischen Erfordernissen.

Für die Abscheidung der Siliciumpulver wurde in einer alternativen Ausführungsform mindestens ein Funktionsmolekül optional ein inertes Verdünnungsgas zur Quenchen im Bereich des Reaktorausgangs des Reaktionszylinders eingesprüht. Über die Temperaturdifferenz und das zugeführte Gasvolumen an Funktionsmolekül kann die Partikelgrösse und die Abscheidungsrate eingestellt werden. Durch Zugabe reaktiver Gase oder organofunktioneller Silane kann hier auch eine Beschichtung, Legierung und/oder Funktionalisierung der Siliciumpulver erfolgen.

Nach der Herstellung über das erfindungsgemäße Verfahren kann das stellvertretend für alle hier beschriebene Zusammensetzungsvarianten stehende Siliciumpulver durch passive Oxidation mit Luft in Reinform einfach gewonnen werden. Alternativ kann bspw. gewonnenes Siliciumcarbid durch Sublimation bei hohen Temperaturen und gegebenenfalls im Hochvakuum, weiter aufgereinigt und/oder abgeschieden werden.

Ebenfalls Gegenstand der Erfindung sind Formulierungen enthaltend Siliciumpulver erhältlich nach dem erfindungsgemäßen und mindestens einen Hilfsstoff und/oder ein Bindemittel und optional eine Lösemittel. Bevorzugte Formulierungen können vorliegen in Form einer Suspension, Dispersion oder eine Einbettung, einer Matrix, einer kosmetischen Formulierung, pharmazeutischen Formulierung, einer Bremspaste, Schleifpaste, Druckpaste, Tinte, in druckbaren Polymeren, elektrisch leitenden Paste, Haftklebemasse, eingebettet in eine Folie oder beispielsweise auch als Gründling vorliegen.

Siliciumpulver steht stellvertretend für alle hier beschriebene Pulver-Zusammensetzungsvarianten.

Das Siliciumpulver kann auch in gepresster, extrudierter, granulierter, gesinterter, kristallisierter Form vorliegen und entsprechend abgepackt werden.

Die Formulierung kann in Form von Presslingen, als kosmetische Formulierung vorliegen. Eine kosmetische Formulierung kann eine Creme, ein Fluid sein, dass optional Siliciumpulver oder oberflächlich mit SiO₂ beschichtete Siliciumpulver als Sonnenschutz-Komponente umfasst. Mit SiO2 beschichtete Siliciumpulver bilden sich durch Oxidation der Siliciumpartikel.

Gegenstand der Erfindung ist auch eine Formulierung enthaltend ein Siliciumpulver, insbesondere Silicium Partikel mit einem Hilfsmittel, Lösungsmittel und/oder Dispersionsmittel und/oder Suspensionsmittel.

Das amorphe Siliziumpulver zeichnet sich durch ein hohes Absorptionsvermögens von Photonen aus. Aufgrund dieser Eigenschaft ist das amorphe Siliciumpulver, insbesondere nano-Silizium, ein besonders geeignetes Funktionsmaterial für Anwendungen die die elektrische Widerstandfunktion in Abhängigkeit von der Bestrahlung nutzen.

So wurde überraschend festgestellt, dass sich das erfindungsgemäße amorphe Siliciumpulver durch eine Photoleitung auszeichnet, die sich bei Abdunkelung reduziert. Die Partikel lassen sich als Schicht prozessieren, wobei die Schicht optisch im Bereich 600-1000 nm Wellenlänge transparent erscheint. Dabei ist es besonders bevorzugt, wenn das Siliciumpulver zur Herstellung von im Wellenlängenbereich von 600 bis 1000 nm transparenten Siliciumschichten verwendet wird, insbesondere ist die Transparenz dieser Schichten größer gleich 90%, vorzugsweise größer gleich 95%, besonders bevorzugt größer gleiche 98%.
Nach einer besonders bevorzugten Ausführungsform wird das amorphe Siliciumpulver verwendet zur Herstellung von Funktionsmaterial, dessen elektrische Widerstandfunktion sich in Abhängigkeit von der Bestrahlung ändert, vorzugsweise zur Herstellung von amorphen Siliciumschichten mit einer Photoleitung.

Die erfindungsgemäßen Siliciumpulver eignen sich aufgrund ihrer Inertheit und ihres Transmissionsverhaltens besonders gut als UV-Schutzmittel, da die erfindungsgemäßen Pulver transparent vorliegen können und vorteilhaft bei 300 nm keine nennenswerte Transmission aufweisen.

Gegenstand der Erfindung ist auch die Verwendung der Siliciumpulver sowie die nach dem Verfahren erhältlichen Siliciumpulver zur Herstellung von Thermoelementen, Halbleitern, Halbleitermaterialien, Solarsilicium, Halbleitersilicium, Pasten, Cremes, Kosmetikprodukten (Sonnenschutzcremes, allergiearmen Kosmetikprodukten), pharmazeutischen Produkten, Silicium enthaltenden Pasten (Solarindustrie, Beschichtung), Elektroden, Membranen, Katalysatoren, von LEDs, Displays, transparenter Schichten, Flat Panel Displays, Schmelztiegeln, Tiegeln zur Kristallisation hochreiner metallischer Verbindungen, Beschichtungen, Aerosolen, Materialien für die Thermoelektrik, zur Herstellung hochreiner polykristalliner Metallverbindungen oder von Einkristallen der Metallverbindungen, in Hochtemperatur Transistoren, die bei 100 bis 400 °C, vorzugsweise um 300 °C betrieben werden können, zur Herstellung von Hochtemperatursperrschichten, die bei 300 bis 400 °C betrieben werden können, Zur Herstellung von Hochtemperaturmembranen, die bei 300 bis 400 °C betrieben werden können, zur Herstellung von Hochtemperaturbauteilen in Batterien, die bei 300 bis 400 °C betrieben werden können, zur Herstellung von Bauteilen, deren Absorption bei 300 bis 1000nm sich im Wesentlichen linear ändert, insbesondere von Bauteilen, deren Transmission bei etwa 1000 nm 50 % beträgt und bis 450 nm oder bis etwa 300 nm 20 % beträgt und ab 300 nm im Wesentlichen keine Transmission mehr erlaubt, oder als Reaktand bei der Herstellung von kristallinem Silicium oder kristallinen Siliciumverbindungen, hochreine Schleifmittel, hochreine Hitzekacheln oder als Material von Artikeln. Ebenfalls Gegenstand der Erfindung ist die Verwendung der Siliciumpulver zur Herstellung von LEDs, Halbleitermaterialien, Flat Panel Displays und zur Herstellung von Materialien für die Thermoelektrik oder zur Herstellung von Einkristallen, Schleifmitteln, Isolatoren, als Feuerfeststoff, wie als Hitzekachel, oder bei der Herstellung von Artikeln oder bei der Herstellung von Elektroden und/oder Schmuck. Verwendung zur Herstellung von Halbleiterausgangsstoffen, Halbleitern, Thermoelementen zur Energierückgewinnung aus Abwärme, insbesondere von hochtemperaturstabilen Thermoelementen sowie zur Herstellung von Hochtemperatursperrschichten und UV/Vis-transparenten und hochtemperaturstabilen Bauteilen verwendet werden.

Die Partikelgrößenbestimmung der Siliciumpulver als auch die Bildung von Agglomeraten oder das Vorliegen von Primärpartikeln kann analytisch mit nachfolgenden Methoden bestimmt werden. Die Bestimmung der Teilchengröße kann u.a. mittels Siebanalyse, TEM (Transelektronenmikroskopie), REM (Rasterkraftelektronenmikroskopie) oder Lichtmikroskopie erfolgen.

Bevorzugt liegt die Leitfähigkeit der Verfahrensprodukte, insbesondere der hochdicht verpreßten pulvrigen Verfahrensprodukte, gemessen zwischen zwei spitzen Elektroden bei κ [m/Ω.m²] = 1 • 10⁻¹ bis 1 • 10⁻⁶. Angestrebt wird für das jeweilige Siliciumpulver eine anwendungsabhägige Leitfähigkeit, die direkt mit der Dotierung bzw. Legierung des Verfahrensproduktes korreliert.

Die nachfolgend erläuterten Figuren und Ausführungsbeispiele erläutern exemplarisch die Gegenstände der Erfindung ohne diese auf diese konkreten Beispiele zu beschränken.

### Ausführungsbeispiele

Die nachfolgenden Figuren stellen dar:
**Figur 1a** Mikrostruktur-Diffusor-Reaktor (in Infrarotstrahlausführung) mit verbesserter Wärmeeinkopplung und Wärmeübergang. Dieser Reaktor kommt vorzugsweise zur Anwendung, um amorphes Silicium darzustellen. Nachfolgend sind die Reaktionsofen Geometriedaten zusammengestellt: **2a:** Reaktor-Rohr: Aussendurchmesser: 36,1 mm; Innendurchmesser: 33,1 mm, **7/7a:** Heizzone Länge 700 mm z.B. bei T = 1150 °C, **15:** Partikelabscheidung, **4.3:** Prozessgaszuführung mit Gasmischeinheit, **AAA** Vorgemischtes Prozessgas, **2.1a:** Reaktionszone z.B. bei 1150°C, **2.3:** Reaktor-Rohr Ausgang (mit Venturi-Trichter) mit Quenchzone, **4.2:** Zuführung Zusatzgas z.B. ABB Wasserstoff/Argon für Regelung Zusammensetzung Prozessgas/ Wärmeübertrag/Partikelbildung im Reaktor,
   **16:** Reaktoreinsatz mit Milli-Kanälen z.B. SiC-Fritte, **AAA:** Prozessgasmischung z.B. Monosilan-Wasserstoff-Gemisch, **16.1:** Millikanal Ausschnitt.
   - **Figur 1b:**: TEM-Aufnahme von amorphem Silicium nach Beispiel 1a (100000 : 1, Auflösung: 200 nm).
   - **Figur 2a:**: TEM-Aufnahme von amorphem Silicium nach Beispiel 1b (200000 : 1, Auflösung: 200 nm).
   - **Figur 2b:**: TEM-Aufnahme von amorphem Silicium nach Beispiel 1b (100000 : 1, Auflösung: 200 nm).
   - **Figur 3a:**: Mikrostruktur-Diffusor-Reaktor (FSR) mit kurzer Verweilzeit sowie verbessertem Wärmeübergang: Anwendungsfeld. Darstellung von amorphen nanoskaligem Si für UV-Stop-Coatings (Temp. typ. 1150°C);
   - **Figur 3b:**: TEM-Aufnahme von nanoscaligem Si mit einem Kristallinitätsindex kleiner 1 %, (200000 : 1, Auslösung: 200 nm).
   - **Figur 4:**: TEM-Aufnahme von amorphen undotierte Silicumpartikeln mit d₅₀ = 17 nm, TEM-Aufnahme: Präparation Ethanol verdünnt aufgetragen, (200000 : 1, Auflösung: 200 nm).

### Bezugszeichenliste:

- **1**: Reaktor 1 (Freiraum-Infrarotstrahlungs- Reaktor, FSR)
- **2**: Reaktionszylinder (horizontal oder vertikal),
- **2a**: horizontaler Reaktionszylinder
- **2.1**: Reaktionsraum; **2.1a:** Reaktionszone, Reaktionszone **2.1a bis 2.1n**
- **2.2**: Reaktorzylinder Ausgang, insbesondere Reaktorrohr Ausgang
- **2.3**: Reaktorzylinder Ausgang (mit Venturi-Trichter) Quenchzone
- **3**: oberhalb 3 im Reaktionsraum **2.1**
- **4**: Abschirmung für Lanze; **4.1:** mindestens einer Düse
- **4.2**: Zuführung tangential, insbesondere für Zirkulargasströmung im Reaktor
- **4.3**: Prozessgaszuführung mit Gasmischeinheit
- **5**: untere Bereich im Reaktor **2.1**
- **6**: Vorrichtung
- **7**: Heizvorrichtung; **7a:** Heizzone
- **8**: Reaktorwand
- **9**: Plasmagenerator
- **10**: gasdurchlässige Membran
- **11**: Düse(n)
- **12**: rotierende Vorrichtung
- **13**: Temperatur-Messlanze
- **14**: Temperatur-Messfühler
- **15**: Partikelabscheidung
- **16**: Reaktoreinsatz mit Milli-Kanälen z.B. SiC-Fritte
- **16.1**: Millikanal Ausschnitt
- **P**: Prozessgas
- **M**: Messpunkte im Reaktor

**A:** Prozessgas A; **B:** Prozessgas B; **AB:** Wasserstoff/Ethan für Regelung Zusammensetzung Prozessgas / Wärmeübertrag/Partikelbildung im Reaktor; **AA:** Prozessgasmischung z.B. Monosilan-Ethan-Gemisch; **AAA:** Vorgemischtes Prozessgas; **ABB** Wasserstoff/Argon für Regelung Zusammensetzung Prozessgas / Wärmeübertrag/Partikelbildung im Reaktor; **AAA** Prozessgasmischung z.B. Monosilan-Wasserstoff-Gemisch.

Alle Angaben in Liter entsprechen Normliter. Der Plasmareaktor wurde bei 0,45 kW und ca. 14 kHz mit Hochspannungspulsen mit einer Halbwertsbreite von t(50) 567ns und einem Massestrom von 43 NL/min betrieben. Für die nachstehend genannten Plasma-Verfahren wurden im nichtthermischen Gleichgewicht betriebene Gasentladungen verwendet.

**Ausführungsbeispiel 1a:** Gewinnung von Siliciumpulvern in hochreiner Form in einem Freiraum-Infrarotstrahlungs- Reaktor durch Zersetzung von Tetraethoxysilan (TEOS).

1 Si(OCH₂CH₃)₄ → Si - Primärpartikel

Es wird Si-Pulver erhalten, das über einen an das Reaktionsrohr angebrachten Konus einem Filter zugeführt wird, in welchem die Siliciumpartikel aufgehalten und isoliert werden. Die Umsetzung erfolgte bei einer Temperatur von ca. 1100 °C mit einer Verweilzeit von 60 Millisekunden in einer H₂-Atmospäre. Erhalten wurde amorphes Silicium mit einer Reinheit größer 85 Gew.-% Silicium.

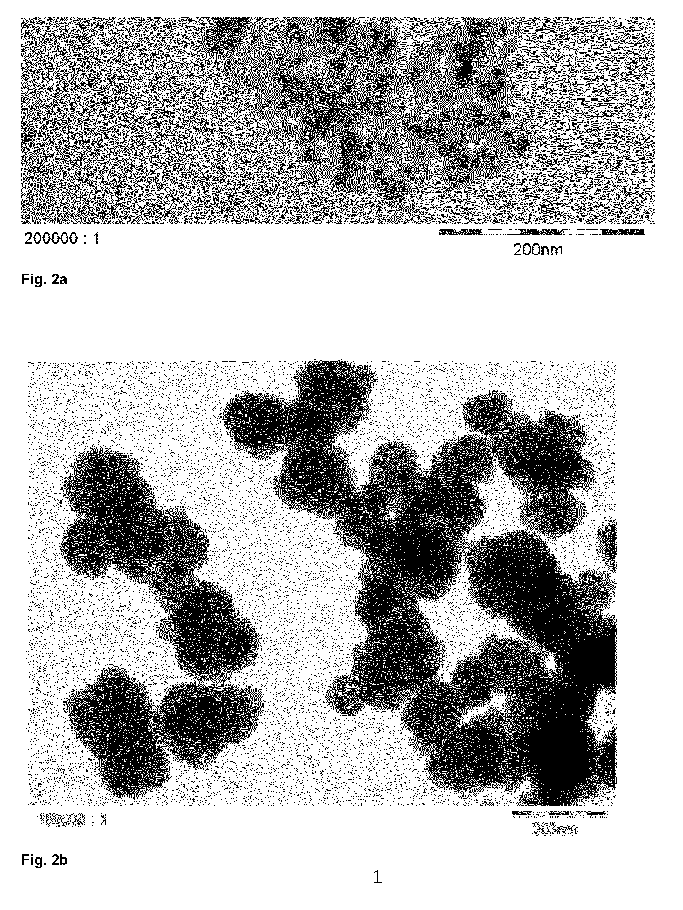

**Ausführungsbeispiel 1b:** Das Verfahren wurde analog dem Ausführungsbeispiel 1a durchgeführt, mit der Abweichung, dass statt TEOS wird Monosilan 5%-ig in Xenonmatrix verwendet wurde. Erhalten wurde amorphes Silicium gemäß **(****Figuren 2a und 2b****).**

**Ausführungsbeispiel 2:** Gewinnung von oberflächenlegierten Siliciumpartikel in hochreiner Form in einem Free-Space Reaktor (FSR). Es wird Monosilan zur Reaktion gebracht. Gemäß der allgemeinen Reaktionsgleichung

1 SiH₄ → Si + 2 H₂

wird Monosilan zersetzt. In der Gasphase, an einer in Richtung Ausgang liegenden Einspeisestelle am FSR wird eine borhaltige Verbindung, z.B. Diboran eingedüst und im einem FSR der **Figur 3a** thermisch zersetzt wodurch Bor auf der Partikeloberfläche abgeschieden wird.

Das freie Reaktorvolumen beträgt ca. 10 ml. Der Reaktor wurde beschickt mit 10 Normliter/ Minute Argon und 5 Normliter/Min Argon mit 5 Gew.-% SiH₄ bei einer Temperatur von 1100 °. Die Verweilzeit betrug 30 Millisekunden (Reaktor **Figur 3a**)**.** Die zweite Einspeisung erfolgt mit 1 Normliter/Min Argon mit 5 Gew.-% SiH₄ und 0,1% Diboran bei einer Temperatur von 1150 °C, wobei die Verweilzeit 20 Millisekunden betrug. Das Produkt wird über einen am Ende des Reaktionsrohr angebrachten Konus einem Filter zugeführt, in welchem die Silicium-Borpartikel aufgehalten werden und isoliert werden.

Erhalten wurde amorphes dotiertes Si mit unterschiedlichem Borgehalt auf der Oberfläche (z.B. 2 Gew-%), hergestellt bei ca. 1100 °C in einer H₂-Atmospäre.

**Ausführungsbeispiel 3:** Gewinnung von amorphen Siliciumpartikeln in hochreiner Form in einem Freiraum-Infrarotstrahlungs- Reaktor. Es wird Monosilan zur Reaktion gebracht. In der Gasphase wird Monosilan im FSR thermisch zersetzt. Der Reaktor wurde beschickt mit 2 Normliter/Min Argon und 1 Normliter/Min Argon mit 5 Gew.-% SiH₄ bei einer Temperatur von 1300 °C und einer Verweilzeit von 30 Millisekunden im Reaktor gemäß **Figur 1a****.** Das Produkt wird über einen am Ende des Reaktionsrohr angebrachten Konus einem Filter zugeführt, in welchem die Silicium-Partikel aufgehalten werden und isoliert werden können.
Amorphes Si wurde bei ca. 1300 °C in Argon-Atmospäre hergestellt.

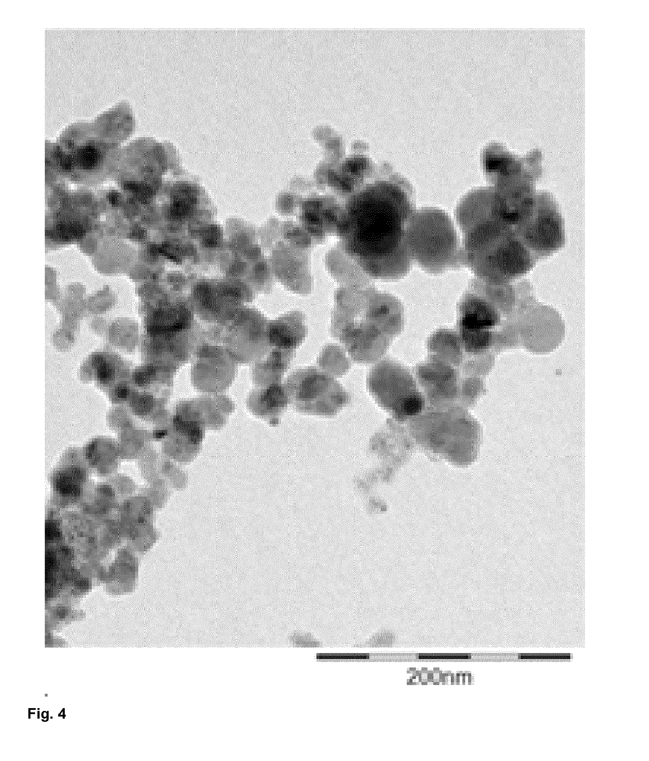

**Ausführungsbeispiel 4:** Im Plasma mit gepulster Spannung von 25 kVpp (Kilovolt Peak-Peak), (die Erzeugung geeigneter Kurvenverläufe kann durch Fourier-Synthese erfolgen) wird Monosilan zersetzt und amorphes Silicium isoliert. **Figur 4** zeigt die TEM-Aufnahme von amorphen, undotierten Silicumpartikeln mit d₅₀ = 17 nm, TEM-Aufnahme: Präparation Ethanol verdünnt aufgetragen.

**Ausführungsbeispiel 5:** Gewinnung von oberflächenlegierten Siliciumpartikeln in hochreiner Form in einem Freiraum Reaktor. Es wird Monosilan zur Reaktion gebracht. Gemäß der allgemeinen Reaktionsgleichung

1 SiH₄ → Si + 2 H₂

wird Silicium abgeschieden. In der Gasphase, an einer in Richtung Ausgang liegenden Einspeisestelle am FSR wird eine borhaltige Verbindung, z.B. Diboran eingedüst und im FSR thermisch zersetzt, wodurch Bor auf der Partikeloberfläche abgeschieden wird. Reaktionsbedingungen: 2 Normliter/Min Argon und 1 Normliter/Min Argon mit 5 Gew.-% SiH₄ werden bei 1100 °C mit einer Verweilzeit von 60 Millisekunden in einem Reaktor umgesetzt.

Das Produkt wird über einen am Ende des Reaktionsrohr angebrachten Konus einem Filter zugeführt, in welchem die Silicium-Borpartikel aufgehalten und isoliert werden.

Amorphes Silicium mit unterschiedlichem Borgehalt auf der Oberfläche (z.B. 2 Gew-%) wurde bei ca. 1100 °C in H₂-Atmosphäre hergestellt.

## Patentansprüche

1. Siliciumpulver, **dadurch gekennzeichnet,**
**dass** die Siliciumpulver Primärpartikel mit einer Partikelgröße von 1 nm bis 100 nm aufweisen und die Partikel im Wesentlichen amorph sind, wobei das Siliciumpulver eine Kristallinität von kleiner gleich 15 % bis 0,1% aufweist.

2. Siliciumpulver nach Anspruch 1, **dadurch gekennzeichnet, dass** das Siliciumpulver eine Photoleitung aufweist.

3. Siliciumpulver nach Anspruch 1 oder 2, **dadurch gekennzeichnet, dass** das Siliciumpulver Primärpartikel mit einer Siliciumlegierung mit größer gleich
10²¹ Atomen/cm³ bis 10²² Atomen/cm³ ausgewählt aus mindestens einem Elektronendonator und Elektronenakzeptor umfasst.

4. Siliciumpulver nach einem der Ansprüche 1 bis 3, **dadurch gekennzeichnet, dass** das Siliciumpulver Primärpartikel und Cluster der Primärpartikel umfasst.

5. Siliciumpulver nach einem der Ansprüche 1 bis 4, **dadurch gekennzeichnet, dass** die Siliciumpulver umfassend Primärpartikel und optional Cluster eine Siliciumlegierung mit einem Element der Gruppe 13 oder 15 des Periodensystems (PSE) der Elemente nach IUPAC aufweisen und/oder zumindest eine Zone einer Siliciumlegierung mit einem Element der Gruppe 13 oder 15 aufweisen.

6. Verfahren zur Herstellung von Siliciumpulver nach einem der Ansprüche 1 bis 5,
indem
a) mindestens eine bei erhöhter Temperatur gasförmige Siliciumverbindung,
b) optional in Gegenwart eines Verdünnungsgases in einer im Wesentlichen sauerstofffreien Atmosphäre unter (i) thermischen Bedingungen und/oder (ii) im Plasma zersetzt wird, und in Form eines Siliciumpulvers als Primärpartikel deren Partikelgröße bei 1 nm bis 100 nm liegt, und optional als Cluster umfassend Primärpartikel erhalten wird, wobei die Partikel im Wesentlichen amorph sind.

7. Verfahren nach Anspruch 6, **dadurch gekennzeichnet, dass** Siliciumpulver umfassend Primärpartikel mit einer Siliciumlegierung mit größer gleich
10²¹ Atomen/cm³ bis 10²² Atomen/cm³ ausgewählt aus mindestens einem Elektronendonator und Elektronenakzeptor erhalten werden.

8. Verfahren nach Anspruch 6 oder 7, **dadurch gekennzeichnet, dass** die bei erhöhter Temperatur gasförmige Siliciumverbindung umfasst Wasserstoff- und/oder Halogen-enthaltende Silane und/oder Kohlenwasserstoff-enthaltende Silane, wie Halogensilane, Chlorsilane, wie Dichlorsilan, Trichlorsilan, Tetrachlorsilan, Hexachlordisilan, Methyltrichlorsilan, Polyhalogensilane sowie reine H-Silane wie Monosilan, Wasserstoff enthaltende Polysilane oder Polyhalogensilane und/oder mindestens ein Alkoxysilan.

9. Verfahren nach einem der Ansprüche 6 bis 8, **dadurch gekennzeichnet, dass** eine gasförmige Siliciumverbindung und optional in Gegenwart eines Verdünnungsgases unter (i) thermischen Bedingungen bei einer Temperatur von 600 bis 1500 °C zersetzt wird.

10. Verfahren nach einem der Ansprüche 6 bis 9, **dadurch gekennzeichnet, dass** das Verdünnungsgas Argon, Helium, Xenon, Krypton, Wasserstoff oder ein Gemisch mindestens zwei der genannten Gase umfasst.

11. Verfahren nach einem der Ansprüche 6 bis 10, **dadurch gekennzeichnet, dass** mindestens ein bei erhöhter Temperatur reaktives Gas umfassend Bor enthaltende Verbindungen, wie Diboran, Stickstoff enthaltende Verbindungen, wie Stickstoff, NH₃, Alkylamine, Germanium enthaltende Verbindungen, Antimon enthaltende Verbindungen, Selen enthaltende Verbindungen, Kohlenwasserstoffe, wie Methan, Butan und/oder Propan, Verbindungen der Elemente der dritten Hauptgruppe des Periodensystems der Elemente (Gruppe 13 oder 15 des Periodensystems der Elemente (PSE) nach IUPAC) und/oder Elementen der fünften Hauptgruppe (Gruppe 15 des Periodensystems der Elemente (PSE) nach IUPAC) zugeführt wird.

12. Verfahren nach einem der Ansprüche 6 bis 11, **dadurch gekennzeichnet, dass** die Verfahrensführung mindestens die Alternativen umfasst
- (A) Zersetzung der Siliciumverbindung in Gegenwart eines Verdünnungsgases und optional mindestens eines reaktiven Gases unter (i) thermischen Bedingungen und/oder (ii) im Plasma, und Ausbildung von Primärpartikeln und/oder Clustern der Primärpartikel, optional umfassend eine Siliciumlegierung, mit den Schritten
- (B) Bildung von amorphen Primärpartikeln umfassend mindestens eine intrinsische Zone einer Siliciumlegierung, und/oder Clustern der Primärpartikel und/oder
- (C) Bildung von reinen, amorphen Siliciumprimärpartikeln und/oder Clustern, wobei die in (B) oder (C) gebildeten Primärpartikeln und/oder Cluster unter (i) thermischen Bedingungen und/oder in (ii) einer Behandlung im Plasma erfolgt und optional
- (D.1) die Partikel aus (B) und (C) unter (i) thermischen Bedingungen und/oder in (ii) einer Behandlung im Plasma mit mindestens einer Zone einer Siliciumlegierung zumindest teilweise beschichtet werden.

13. Verfahren nach einem der Ansprüche 6 bis 12, wobei die Herstellung des Siliciumpulvers umfasst mindestens den Schritt Zersetzung (A) der Siliciumverbindung optional in Gegenwart des Verdünnungsgases unter
(i) thermischen Bedingungen und/oder (ii) im Plasma, und Ausbildung von Primärpartikeln und/oder Clustern der Primärpartikel innerhalb von 10 bis 1000 Millisekunden.

14. Siliciumpulver erhältlich nach einem Verfahren nach einem der Ansprüche 6 bis 13, **dadurch gekennzeichnet, dass** die Siliciumpulver umfassend im Wesentlichen amorphe Primärpartikel, wobei das Siliciumpulver eine Kristallinität von kleiner gleich 15 % bis 0,1% aufweist.

15. Reaktor (Freiraum-Infrarotstrahlungs-Reaktors) (1) zur Herstellung von Siliciumpulvern nach einem der Ansprüche 1 bis 5 oder einem Verfahren der Ansprüche 6 bis 13, mit Partikeln deren Primärpartikelgröße von 1 nm bis 100 nm liegt, wobei der Reaktor (1) einen Reaktionszylinder (2) mit Reaktionsraum (2.1) umfasst und im Bereich des Reaktoranfangs (2.a, 3) mindestens eine Zuführung (4.1, 4.2, 4.3) aufweist für die Einleitung a) der mindestens einen bei erhöhter Temperatur gasförmigen Siliciumverbindung, und b) optional des Verdünnungsgases, in den Reaktionsraum (2.1), wobei dem Reaktionsraum (2.1) zumindest in einem Bereich am äußeren Umfang des Reaktionsraumes (2.1) Heizvorrichtungen (7) zugeordnet sind, und/oder dem Reaktionsraum (2.1) mindestens ein Plasmagenerator (9) zugeordnet ist, zur Erzeugung eines Plasmas im Reaktionsraum (2.1).

16. Verwendung der Siliciumpulver nach einem der Ansprüche 1 bis 5 oder erhältlich nach einem Verfahren nach einem der Ansprüche 6 bis 13, zur Herstellung von Solarsilicium, Halbleitersilicium, Thermoelementen, Halbleitern, Halbleitermaterialien, Pasten, Cremes, Kosmetikprodukten, pharmazeutischen Produkten, Silicium enthaltenden Pasten, Elektroden, Membranen, Katalysatoren, von LEDs, Displays, transparenter Schichten, Flat Panel Displays, Schmelztiegeln, Tiegeln, als Ausgangsverbindung zur Kristallisation hochreiner metallischer Silicium-Verbindungen, Beschichtungen, Aerosolen, Materialien für die Thermoelektrik, als Ausgangsverbindung zur Herstellung hochreiner polykristalliner Metallverbindungen oder von Einkristallen der Metallverbindungen, in Hochtemperatur Transistoren, die von 100 bis 400 °C, vorzugsweise um 300 °C betrieben werden können, zur Herstellung von Hochtemperatursperrschichten, die bei 300 bis 400 °C betrieben werden können, zur Herstellung von Hochtemperaturmembranen, die bei 300 bis 400 °C betrieben werden können, zur Herstellung von Hochtemperaturbauteilen in Batterien, die bei 300 bis 400 °C betrieben werden können, zur Herstellung von Bauteilen, deren Absorption von 300 bis 1000 nm sich im Wesentlichen linear ändert, insbesondere von Bauteilen, deren Transmission bei etwa 1000 nm 50% beträgt und bis 450 nm oder bis etwa 300 nm 20% beträgt und ab 300 nm im Wesentlichen keine Transmission mehr erlaubt, oder als Reaktand bei der Herstellung von kristallinem Silicium oder kristallinen Siliciumverbindungen, hochreine Schleifmittel, hochreine Hitzekacheln oder als Material von Artikeln oder zur Herstellung von im Wellenlängenbereich von 600 bis 1000 nm transparenten Siliciumschichten, insbesondere ist die Transparenz größer gleich 90%, vorzugsweise größer gleich 95%, besonders bevorzugt größer gleiche 98%, und/oder zur Verwendung als Funktionsmaterial oder zur Herstellung von Funktionsmaterial dessen elektrische Widerstandfunktion sich in Abhängigkeit von der Bestrahlung ändert, vorzugsweise zur Herstellung von amorphen Siliciumschichten, die eine Photoleitung aufweisen.
